# EUROPÄISCHE PATENTSCHRIFT

(11) **EP 3 626 176 B1**
(45) Veröffentlichungstag und Bekanntmachung des Hinweises auf die Patenterteilung: **30.12.2020**
(21) Anmeldenummer: 18195330.8
(22) Anmeldetag: 19.09.2018
(51) Int. Cl.: A61B 6/12

(54) **VERFAHREN ZUM UNTERSTÜTZEN EINES ANWENDERS, COMPUTERPROGRAMMPRODUKT, DATENTRÄGER UND BILDGEBENDES SYSTEM**
METHOD FOR SUPPORTING A USER, COMPUTER PROGRAM PRODUCT, DATA CARRIER AND IMAGING SYSTEM
PROCÉDÉ D'ASSISTANCE D'UN UTILISATEUR, PRODUIT PROGRAMME INFORMATIQUE, SUPPORT DE DONNÉES ET SYSTÈME D'IMAGERIE

(43) Veröffentlichungstag der Anmeldung: 25.03.2020
(73) Patentinhaber: Siemens Healthcare GmbH, 91052 Erlangen (DE)
(72) Erfinder: Regensburger, Alois, 91058 Erlangen (DE)

(56) Entgegenhaltungen:
- WO-A1-2008/120136
- WO-A1-2016/182550
- DE-B3-102016 202 512
- US-A1- 2015 030 229
- US-A1- 2015 085 981
- US-A1- 2016 260 220

## Beschreibung

Die Erfindung betrifft ein Verfahren zum Unterstützen eines Anwenders, ein entsprechendes Computerprogrammprodukt, einen entsprechenden Datenträger und ein entsprechendes bildgebendes System.

Es gibt heutzutage eine Vielzahl unterschiedlicher Bildgebungsmodalitäten, welche bevorzugt beispielsweise in einem medizinischen Umfeld aber ebenso auch in anderen industriellen Feldern eingesetzt werden, um ein jeweiliges Ziel- oder Untersuchungsobjekt abzubilden. Dabei ist es heutzutage eine verbreitete Technik, Daten aus unterschiedlichen Quellen miteinander zu kombinieren, wobei die entsprechenden Daten, also beispielsweise Bilder oder Datensätze, miteinander registriert werden, um eine ortsgetreue Überlagerung in einem gemeinsamen Koordinatensystem zu ermöglichen. Um eine jeweilige Situation möglichst genau und zuverlässig erkennen und bewerten zu können oder um beispielsweise das abgebildete Untersuchungsobjekt möglichst genau und zuverlässig manipulieren zu können, ist eine möglichst hohe Präzision dieser Registrierung wünschenswert. Fehler, Ungenauigkeiten oder Unsicherheiten bei der Registrierung können sich dementsprechend nachteilig auswirken. Als Beispiel für ein solches Registrierung sei das Verfahren in der US2016/260220 A1 genannt.

Aufgabe der vorliegenden Erfindung ist es, einen Anwender in Situationen mit einer fehlerbehafteten oder unsicheren Registrierung zu unterstützen. Diese Aufgabe wird erfindungsgemäß durch die Gegenstände der unabhängigen Patentansprüche gelöst. Vorteilhafte Ausgestaltungen und Weiterbildungen der vorliegenden Erfindung sind in den abhängigen Patentansprüchen, in der Beschreibung und in den Zeichnungen angegeben. Ein erfindungsgemäßes Verfahren dient zum Unterstützen eines Anwenders, beispielsweise bei einem Untersuchen eines Untersuchungs- oder Zielobjekts. Bei dem Verfahren wird zunächst ein das Zielobjekt abbildender 3D-Datensatz bereitgestellt. Dieses Bereitstellen kann dabei etwa ein Bereitstellen oder Verfügbarmachen eines Datenspeichers, auf welchem der 3D-Datensatz gespeichert ist, umfassen oder bedeuten. Ebenso kann das Bereitstellen ein Erfassen oder Aufnehmen, also Messen, des 3D-Datensatzes bedeuten oder umfassen.

Der 3D-Datensatz kann insbesondere mittels einer ersten Bildgebungsmodalität, beispielsweise mittels eines Computertomographen, also eines CT-Röntgengeräts, einer Magnetresonanzanlage (MR-Anlage) oder dergleichen aufgenommen werden oder aufgenommen sein.

Das Zielobjekt kann beispielsweise ein Patient, ein Teil eines Patienten, ein bestimmtes Organ oder eine Gewebeprobe aber ebenso ein letztlich beliebiges mittels einer entsprechenden Bildgebungsmodalität abbildbares Objekt oder Material sein. Es ist beispielsweise bekannt, dass in industriellen Anwendungsgebieten technische Geräte mittels Röntgenstrahlung abgebildet und untersucht werden. Der 3D-Datensatz kann ein Satz, also eine Sammlung von Messwerten oder Datenpunkten oder beispielsweise ein 3D-Bild sein.

Weiter wird bei dem erfindungsgemäßen Verfahren wenigstens ein 2D-Bild des Zielobjekts erfasst. Dieses Erfassen kann beispielsweise ein Abrufen des 2D-Bildes, also entsprechende Daten, aus einem bereitgestellten Datenspeicher aber ebenso ein Messen oder Aufnehmen des 2D-Bildes umfassen oder bedeuten. Das Aufnehmen des 2D-Bildes kann beispielsweise automatisch oder etwa ausgelöst durch ein Betätigen eines Fußschalters oder dergleichen erfolgen. Das 2D-Bild kann insbesondere mittels einer zweiten Bildgebungsmodalität aufgenommen werden oder aufgenommen sein. Die zweite Bildgebungsmodalität kann dabei der ersten Bildgebungsmodalität entsprechen oder von dieser verschieden, insbesondere unterschiedlich, sein. Bevorzugt kann die erste Bildgebungsmodalität ein 3D-Röntgengerät und die zweite Bildgebungsmodalität ein 2D-Röntgengerät oder das in einem 2D-Betrieb betriebene 3D-Röntgengerät sein.

Weiter wird bei dem erfindungsgemäßen Verfahren automatisch eine 2D/3D-Registrierung des wenigstens einen 2D-Bildes mit dem bereitgestellten 3D-Datensatz durchgeführt. Mit anderen Worten werden also das 2D-Bild und der 3D-Datensatz ortsgetreu miteinander kombiniert oder einander überlagert, also in einem gemeinsamen Koordinatensystem derart angeordnet, dass jeweilige einander entsprechende Teile oder Bereiche des abgebildeten Zielobjekts in dem 2D-Bild und dem 3D-Datensatz in einer entsprechenden Überlagerung einander überlappen. Das 2D-Bild kann dabei in drei Raumrichtungen oder räumlichen Dimensionen, welche im Folgenden auch als x, y, und z bezeichnet werden, angeordnet werden. Weiter wird bei dem erfindungsgemäßen Verfahren automatisch eine dieser Raumrichtungen bestimmt, in welcher die 2D/3D-Registrierung eine größte Unsicherheit oder Ungenauigkeit, also einen größten Fehler, aufweist. In der bestimmten Raumrichtung ist die Unsicherheit oder Ungenauigkeit der Registrierung also größer als in den beiden andere Raumrichtungen.

Bekannte Methoden zur 2D/3D-Registrierung sind oftmals nicht in allen drei Raumrichtungen gleich präzise. Insbesondere ist häufig zu beobachten, dass die 2D/3D-Registrierung in zwei orthogonalen Raumrichtungen, beispielsweise x und y, mit relativ hoher Präzision oder Genauigkeit erfolgt, in der dritten Raumrichtung, also in z-Richtung, aber nur relativ ungenau, also mit einer signifikant geringeren Präzision oder Genauigkeit durchgeführt werden kann. Dies kann insbesondere dann auftreten oder der Fall sein, wenn eine Aufnahmerichtung des 2D-Bildes, also beispielsweise eine entsprechende Strahlungs- oder Projektionsrichtung des Röntgengeräts, entlang der z-Richtung oder z-Achse erfolgt. Mit anderen Worten kann also als die Raumrichtung, in welcher die 2D/3D-Registrierung ihre größte Unsicherheit aufweist, die Aufnahmerichtung des 2D-Bildes bestimmt werden. Die Unsicherheit oder Ungenauigkeit der 2D/3D-Registrierung beruht letztlich also auf einer relativ geringen oder mangelnden Tiefenauflösung des 2D-Bildes. Die Raumrichtung der größten Unsicherheit der 2D/3D-Registrierung, also der größten Registrierungsunsicherheit, kann dabei beispielsweise über ein Erkennen oder Nachverfolgen einer Pose des zum Aufnehmen des 2D-Bildes verwendeten Röntgengeräts und des Zielobjekts erfolgen. Aus entsprechenden Daten kann dann direkt oder beispielsweise mittels eines entsprechenden Objekterkennungs- oder Bildverarbeitungsalgorithmus, gegebenenfalls unter Berücksichtigung weiterer Sensordaten und/oder Benutzereingaben, die Aufnahmerichtung für das 2D-Bild, also beispielsweise ein Winkel einer entsprechenden Achse in dem gemeinsamen Koordinatensystem oder in einem Koordinatensystem des 3D-Datensatzes, automatisch bestimmt werden.

Weiter wird bei dem erfindungsgemäßen Verfahren ein Signal zum Ausrichten oder zum Unterstützen eines Ausrichtens eines zum Untersuchen des Zielobjekts vorgesehenen Instruments erzeugt und ausgegeben in Abhängigkeit von der bestimmten Raumrichtung größter Unsicherheit zum Unterstützen des Anwenders. Der Anwender kann also beispielsweise eine Bedienperson des Röntgengeräts und/oder des Instruments, beispielsweise ein Arzt oder ein Techniker, sein. Das Ausrichten des Instruments oder das Unterstützen des Ausrichtens kann bedeuten, dass dabei oder dazu das Instrument selbst bewegt oder verstellt werden kann. Ebenso kann das Ausrichten bedeuten, dass ein anderes Objekt, beispielsweise ein Bildgebungsgerät, ein Patiententisch, oder dergleichen bewegt oder verstellt werden kann, wodurch sich eine neue oder angepasste Relativausrichtung des Instruments, also dessen Ausrichtung relativ zu dem tatsächlich bewegten oder verstellten Objekt, ergibt. Im Folgenden meint das "Ausrichten" also ein direktes oder relatives Ausrichten oder gegebenenfalls ebenso das entsprechende Unterstützen des Ausrichtens.

Das Instrument kann beispielsweise eine Kamera, eine Sonde, eine Nadel, ein Messfühler oder eines einer Vielzahl ähnlieher oder weiterer Instrumente oder Geräte sein. Auf einige Beispiele hierzu wird weiter unten noch näher eingegangen.

Das Signal kann beispielsweise ein in akustischer, optischer und/oder haptischer Hinweis, eine grafische Darstellung, ein Datensignal oder Datensatz, ein Steuersignal und/oder dergleichen mehr sein. Dies wird ebenfalls weiter unten noch näher erläutert. Mittels des Signals oder durch das Signal werden letztlich also dem Anwender Daten oder Informationen bereitgestellt und/oder eine Maßnahme oder ein Vorgang eingeleitet, wodurch dem Anwender vorteilhaft insbesondere eine genauere und/oder zuverlässigere Bewertung einer jeweiligen Situation, also des Zielobjekts, ermöglicht und/oder der Anwender zumindest über die Unsicherheit der 2D/3D-Registrierung und damit gegebenenfalls einer entsprechenden Ausrichtung, Positionierung und/oder Wirkung des Instruments in der bestimmten Raumrichtung informiert wird.

Bei bisherigen Anwendungen wird eine entsprechende Registrierungsunsicherheit oftmals schlichtweg als unvermeidlich in Kauf genommen. Alternativ werden beispielsweise während einer Untersuchung des Zielobjekts statt des 2D-Bildes eine Vielzahl weiterer Bilder oder Abbildungen oder Daten des Zielobjekts aufgenommen, um genügend Daten für eine genauere Registrierung in allen Raumrichtungen zu erhalten. Dadurch können sich jedoch nachteilig eine höhere Dosis oder Belastung des Zielobjekts ebenso wie eine zeitliche Verzögerung und/oder logistische Schwierigkeiten oder Kollisionsprobleme in einem Arbeitsablauf (englisch: Workflow) bei dem Abbilden oder Untersuchen des Zielobjekts ergeben. Zudem wird gegebenenfalls nicht nur zum Aufnehmen des 3D-Datensatzes, sondern ebenso bei dem Untersuchen des Zielobjekts ein entsprechend aufwändigeres und damit teureres bildgebendes System oder Bildgebungssystem, also beispielsweise ein entsprechendes Röntgengerät, benötigt. Zudem birgt eine Registrierungsunsicherheit, welche dem jeweiligen Anwender nicht bewusst oder bekannt ist, eine Gefahr von Fehlentscheidungen, welche ein Ergebnis der jeweiligen Untersuchung des Zielobjekts wertlos machen oder infrage stellen und/oder das Zielobjekt verletzen oder beschädigen können. Diese Nachteile können durch die vorliegende Erfindung vermieden oder abgeschwächt werden.

In vorteilhafter Ausgestaltung der vorliegenden Erfindung wird als das Instrument ein Endoskop, also eine Kamera oder ein bildaufnehmendes Gerät, insbesondere ein Laparoskop, verwendet. Eine Pose, also eine Position und Orientierung, des Endoskops in einem Koordinatensystem des 3D-Bildes und/oder des 2D-Bildes wird dann, gegebenenfalls bis auf eine vorgegebene, also bekannte, Koordinatentransformation, automatisch nachverfolgt. Wird also zum Aufnehmen des 2D-Bildes und zum Positionieren des Endoskops nicht dasselbe Koordinatensystem verwendet, so stehen die beiden Koordinatensysteme also zumindest in einer vorgegebenen räumlichen Lagebeziehung zueinander. Dies kann beispielsweise durch eine entsprechende Messung oder Kalibrierung im Vorhinein sichergestellt werden. Aus wenigstens einem mittels des Endoskops aufgenommenen Endoskopbild des Zielobjekts und dem 3D-Datensatz wird dann wenigstens ein Überlagerungsbild erzeugt. Beispielsweise dann, wenn ein Stereo-Endoskop verwendet wird, können entsprechend zwei Endoskopbilder, nämlich die beiden Teil-Stereobilder, verwendet und entsprechend beispielsweise zwei Überlagerungsbilder erzeugt werden.

Da zwischen dem Aufnehmen des 3D-Datensatzes und dem Aufnehmen des Endoskopbilds eine signifikante Zeitspanne, beispielsweise mehrere Stunden oder mehrere Tage, liegen kann und sich das Zielobjekt dementsprechend zwischenzeitlich in unbekannter Weise bewegt haben kann, wird zum ortsgetreuen Erzeugen des Überlagerungsbildes, also zum ortsgetreuen Überlagern des Endoskopbildes und des 3D-Datensatzes die 2D/3D-Registrierung verwendet. Anhand des 2D-Bildes kann also eine jeweils aktuelle Pose des Zielobjekts bestimmt und in Beziehung zu dem 3D-Datensatzes oder einem Koordinatensystem des 3D-Datensatzes gesetzt werden. Über die bekannte Lagebeziehung der Koordinatensysteme des 2D-Bildes und des Endoskops oder Endoskopbildes kann dann entsprechend eine Lagebeziehung zwischen dem Endoskopbild und dem 3D-Datensatz beziehungsweise eine Lagebeziehung der entsprechenden Koordinatensysteme bestimmt werden.

Für das Überlagerungsbild können dabei das Endoskopbild und der 3D-Datensatz selbst einander überlagert werden. Ebenso kann das Endoskopbild jedoch mit einer aus dem 3D-Datensatz erzeugten oder abgeleiteten Darstellung oder Struktur, beispielsweise einem virtuellen Modell oder etwa einem Segmentierungsgitter oder einer segmentierte Darstellung, kombiniert, also überlagert werden. Das Endoskopbild kann also insbesondere ein Kamerabild oder ein Video sein, welches das Zielobjekt optisch realistisch, also realitätsgetreu, abbildet. Das Überlagerungsbild kann dementsprechend als augmentierte Realität (englisch: Augmented Reality) aufgefasst oder erzeugt werden.

Als das Signal oder als Teil des Signals wird dann ein Hinweis an den Anwender ausgegeben, dass und wie er die Ausrichtung des Endoskops verändern kann, sodass sich eine Erfassungsrichtung des Endoskops zumindest im Wesentlichen entlang der bestimmten Raumrichtung der größten Registrierungsunsicherheit erstreckt, um einen durch die Unsicherheit der 2D/3D-Registrierung verursachten Visualisierungsfehler in dem Überlagerungsbild zu verringern.

Die Erfassungsrichtung des Endoskops entspricht dabei einer optischen Achse des Endoskops und derjenigen Richtung, in oder entlang welcher beispielsweise ein Zentralstrahl eines von dem Endoskop erfassten Lichtbündels erfasst wird. Die Erfassungsrichtung des Endoskops kann also insbesondere orthogonal zu einer Bildebene des Endoskopbilds sein. Das Ausrichten des Endoskops beziehungsweise der Erfassungsrichtung des Endoskops entlang der bestimmten Raumrichtung kann dabei eine zumindest im Wesentlichen parallele oder antiparallele Ausrichtung bedeuten.

Insbesondere dann, wenn nur ein 2D-Bild für die 2D/3D-Registrierung verwendet wird, kann die Erfassungsrichtung des Endoskops also entlang der Aufnahmerichtung dieses einen 2D-Bildes ausgerichtet werden.

Das Ausrichten des Endoskops beziehungsweise von dessen Erfassungsrichtung zumindest im Wesentlichen entlang der bestimmten Raumrichtung kann dabei ein entsprechendes Ausrichten bis auf Fehler oder Abweichungen von beispielsweise 10° bedeuten. Die bestimmte Raumrichtung der größten Unsicherheit der 2D/3D-Registrierung fällt dann also zumindest im Wesentlichen mit einer Betrachtungs-, oder Tiefenrichtung des Überlagerungsbildes zusammen. Die Unsicherheit kann dann zwar weiterhin bestehen, wird erfahrungsgemäß bei dieser Ausrichtung von einem Betrachter aber als wenig störend wahrgenommen und kann daher toleriert werden.

Mittels des Signals oder als das Signal kann also beispielsweise eine grafische Hilfestellung angezeigt werden, welche eine aktuelle Winkelabweichung zwischen der Erfassungsrichtung des Endoskops und der bestimmten Raumrichtung angibt. Weiter kann mittels des Signals, als das Signal oder als Teil des Signals eine Hilfestellung angezeigt oder ausgegeben werden, um den Anwender bei dem Ausrichten des Endoskops zu unterstützen. Dies kann beispielsweise über entsprechende Pfeile, eine Farbkodierung und/oder eine Fadenkreuzansicht (englisch: Bulls-Eye View) realisiert werden. Entsprechende Anzeigen oder Darstellungen können auf einem entsprechenden Anzeigegerät, beispielsweise einem Monitor oder einem Head-Mounted Display (HMD), also einer an einem Kopf des jeweiligen Betrachters oder Anwenders befestigten Anzeige, angezeigt, also ausgegeben werden.

Es ist dabei zu beachten, dass die Erfassungsrichtung des Endoskops nicht notwendigerweise mit einer Zentral- oder Längsachse des Endoskops selbst, also dessen Gehäuse oder Korpus, übereinstimmen muss. Eine entsprechende Winkelabweichung kann jedoch als Parameter oder Parameterwert vorgegeben werden.

Insgesamt kann so eine größere tatsächliche oder zumindest wahrgenommene Präzision des Überlagerungsbildes beziehungsweise der entsprechenden Überlagerung in Augmented-Reality-Anwendungen (AR-Anwendungen) erreicht werden, ohne dass hierfür zusätzliche Bilder oder Aufnahmen des Zielobjekts, beispielsweise weitere Röntgenaufnahmen für die Registrierung mit dem 3D-Datensatz notwendig sind. Es genügt eine entsprechende Ausrichtung des Endoskops, welche einfach, schnell, flexibel und ohne Belastung des Zielobjekts durchgeführt, also erreicht werden kann. Somit kann auf besonders einfache und schonende Weise ein Risiko für Fehlentscheidungen aufgrund einer dem jeweiligen Anwender nicht bewussten oder bekannten Unsicherheit in einer Raumrichtung bei der 2D/3D-Registrierung vermindert werden. Es ist beispielsweise verständlich und nachvollziehbar, wenn ein Anwender davon ausgeht, dass die gegebenenfalls offenkundig hohe Präzision oder Genauigkeit der Registrierung in x- und y-Richtung entsprechend auch für die z-Richtung gilt, auch wenn dies tatsächlich nicht der Fall ist.

In weiterer vorteilhafter Ausgestaltung der vorliegenden Erfindung wird als das Instrument ein mittels eines Roboters geführtes Endoskop verwendet. Auch hier wird eine Pose des Endoskops in einem Koordinatensystem des 3D-Bildes und/oder des 2D-Bildes, zumindest bis auf eine vorgegebene Koordinatentransformation, automatisch nachverfolgt (getrackt, von englisch: "instrument tracking"). Auch hier wird aus einem mittels des Endoskops aufgenommenen Endoskopbild des Zielobjekts und dem 3D-Datensatz ein Überlagerungsbild erzeugt. Als das Signal oder als Teil des Signals wird dann ein Steuersignal für den Roboter erzeugt, durch welches der Roboter das Endoskop automatisch ausrichtet, sodass dessen Erfassungsrichtung sich entlang der bestimmten Raumrichtung erstreckt, um einen durch die Unsicherheit der 2D/3D-Registrierung verursachten Visualisierungsfehler in dem Überlagerungsbild zu verringern.

Mit anderen Worten wird also das robotergeführte Endoskop automatisch für eine optimierte Visualisierung oder Bildqualität des Überlagerungsbildes ausgerichtet, insbesondere nachgeführt. Dazu kann zusätzlich beispielsweise eine aktuelle Pose des Zielobjekts überwacht und nachverfolgt und bei dem Ausrichten des Endoskops, also bei dem entsprechenden Erzeugen des Steuersignals automatisch berücksichtigt werden. Auf diese Weise kann das Endoskop besonders schnell, präzise und zuverlässig entsprechend ausgerichtet werden. Somit kann besonders genau und zuverlässig eine optimale Visualisierungsqualität des Überlagerungsbildes sichergestellt werden. Besonders vorteilhaft muss dabei der jeweilige Anwender hierauf keine Aufmerksamkeit oder Mühe verwenden, sodass die Untersuchung des Zielobjekts gegebenenfalls schneller und mit weniger Ablenkungen oder weniger Belastung für den jeweiligen Anwender durchgeführt werden kann.

In weiterer vorteilhafter Ausgestaltung der vorliegenden Erfindung wird ebenfalls als das Instrument ein Endoskop verwendet und dessen Pose in einem Koordinatensystem des 3D-Bildes und/oder des 2D-Bildes, zumindest bis auf eine vorgegebene Koordinatentransformation, nachverfolgt. Auch hier wird aus einem mittels des Endoskops aufgenommenen Endoskopbild des Zielobjekts und dem 3D-Datensatz ein Überlagerungsbild (AR-Bild) erzeugt. Als das Signal oder als Teil des Signals wird dann ein Steuersignal für eine zum Aufnehmen des 2D-Bildes verwendete Bildgebungsmodalität, also für die genannte zweite Bildgebungsmodalität, beispielsweise für ein C-Bogen-Röntgengerät, erzeugt. Durch dieses Steuersignal wird die Bildgebungsmodalität, also das Röntgengerät, automatisch so ausgerichtet, dass sich deren beziehungsweise dessen Abbildungs- oder Aufnahmerichtung automatisch entlang einer Erfassungsrichtung des Endoskops erstreckt, um einen durch die Unsicherheit der 2D/3D-Registrierung verursachten Visualisierungsfehler in dem Überlagerungsbild zu verringern.

Mit anderen Worten kann also abhängig von der Registrierungsunsicherheit und der aktuellen Pose, insbesondere Orientierung, des Endoskops und dessen Erfassungsrichtung automatisch eine Angulation der zweiten Bildgebungsmodalität, insbesondere also des Röntgengeräts, angepasst oder nachgeführt werden. Die zweite Bildgebungsmodalität wird also als Hilfestellung für den jeweiligen Anwender automatisch derart verfahren oder eingestellt, dass deren Aufnahmerichtung, also insbesondere eine entsprechende Röntgen-Projektionsrichtung, sich parallel oder antiparallel zu einer Blickrichtung, also der Erfassungsrichtung des Endoskops erstreckt. Auch hierdurch kann vorteilhaft der Visualisierungsfehler in dem Überlagerungsbild besonders genau, schnell und zuverlässig minimiert und der Anwender entlastet werden.

In weiterer vorteilhafter Ausgestaltung der vorliegenden Erfindung wird ebenfalls als das Instrument ein Endoskop verwendet und dessen Pose in einem Koordinatensystem des 3D-Bildes und/oder des 2D-Bildes, zumindest bis auf eine vorgegebene Koordinatentransformation, nachverfolgt. Auch hier wird aus einem mittels des Endoskops aufgenommenen Endoskopbild des Zielobjekts und dem 3D-Datensatz ein Überlagerungsbild erzeugt. Als das Signal oder als Teil des Signals wird dann eine Visualisierung des Überlagerungsbildes ortsabhängig gemäß ein Grad der Unsicherheit der 2D/3D-Registrierung angepasst, insbesondere durch Unscharfzeichnen, Ausgrauen, Strecken oder Verzerren und/oder Ausblenden eines Teils des Überlagerungsbildes. Aufgrund der Unsicherheit der 2D/3D-Registrierung können also auch das Endoskopbild und der 3D-Datensatz nicht hundertprozentig genau oder mit hundertprozentiger Sicherheit ortsgenaue überlagert oder kombiniert werden. Diese Unsicherheit oder Ungenauigkeit bei dem Überlagern, also in dem Überlagerungsbild, kann dabei an verschiedenen Stellen des Überlagerungsbildes, also ortsabhängig, unterschiedlich sein. Eine Unsicherheit der Überlagerung oder des Überlagerungsbildes kann dabei insbesondere dynamisch abhängig sein von einer relativen Ausrichtung des Endoskops beziehungsweise der Erfassungsrichtung des Endoskops in Bezug auf die bestimmte Raumrichtung der größten Unsicherheit der 2D/3D-Registrierung.

Weicht die Erfassungsrichtung des Endoskops also beispielsweise um einen Winkel α von der bestimmten Raumrichtung ab, so kann das Überlagerungsbild entsprechend angepasst, also grafisch bearbeitet, werden, um dem jeweiligen Betrachter oder Anwender den Grad der Unsicherheit oder Ungenauigkeit, also eines Visualisierungsfehlers, zu signalisieren, also anzuzeigen. Das Unscharfzeichnen kann dann bevorzugt beispielsweise proportional zu sinus(α) erfolgen. Ebenso können beispielsweise Pfeile oder andere Symbole die Winkelabweichung und/oder die bestimmte Raumrichtung anzeigen. Besonders bevorzugt werden nur der 3D-Datensatz oder auf dem 3D-Datensatz beruhende Anteile des Überlagerungsbildes entsprechend angepasst. Das Endoskopbild bleibt als Teil des Überlagerungsbildes also dann beispielsweise unverändert und es werden nur diesem überlagerte eingeblendete Überlagerungsstrukturen angepasst oder modifiziert. Besonders bevorzugt kann ein Schwellenwert für die Abweichung der Winkelabweichung, also den Winkel α zwischen der Erfassungsrichtung des Endoskops und der bestimmten Raumrichtung, vorgegeben sein. Wird dieser Schwellenwert oder Schwellenwertwinkel erreicht oder überschritten, so können die Überlagerungsstrukturen, also auf dem 3D-Datensatz beruhende Anteile des Überlagerungsbildes, komplett ausgeblendet werden. Auf diese Weise kann besonders intuitiv und anschaulich, also besonders einfach verständlich und damit besonders zuverlässig der jeweilige Betrachter oder Anwender auf die Unsicherheit der Darstellung aufmerksam gemacht werden.

In vorteilhafter Weiterbildung der vorliegenden Erfindung wird die Visualisierung des Überlagerungsbildes nur in der bestimmten Raumrichtung der größten Unsicherheit der 2D/3D-Registrierung angepasst. Mit anderen Worten erfolgt beispielsweise das Unscharfzeichnen der Überlagerungsstrukturen nur in oder entlang dieser Raumrichtung, also in z-Richtung. Ebenso können die Überlagerungsstrukturen beispielsweise nur in z-Richtung gestreckt werden, also gestreckt oder verzerrt dargestellt werden, um die Unsicherheit, also einen resultierenden Visualisierungsfehler oder eine resultierende Visualisierungsunsicherheit in dem Überlagerungsbild, zu repräsentieren oder anzuzeigen. Auf diese Weise kann dem jeweiligen Betrachter oder Anwender besonders einfach und besonders genau die Problematik angezeigt und vermittelt werden, dass die 2D/3D-Registrierung in der bestimmten Raumrichtung besonders unsicher oder besonders ungenau ist. Es kann dabei vorteilhaft beispielsweise auf zusätzliche Markierungen, Einblendungen oder Symbole verzichtet werden, wodurch eine Ablenkung oder Beanspruchung des Anwenders oder Betrachters verringert oder vermieden werden kann.

In weiterer vorteilhafter Ausgestaltung der vorliegenden Erfindung wird als das Instrument ein Stereo-Endoskop verwendet. Eine Pose des Stereo-Endoskops in einem Koordinatensystem des 3D-Bildes und/oder des 2D-Bildes wird dann, zumindest bis auf eine vorgegebene Koordinatentransformation, automatisch nachverfolgt. Auch hier wird aus einem mittels des Stereo-Endoskops aufgenommenen stereoskopischen Endoskopbild des Zielobjekts und dem 3D-Datensatz ein Überlagerungsbild erzeugt. Dabei ist es vorgesehen, dass mittels einer Vergenz oder Parallaxe zwischen einem linken Teilbild und einem rechten Teilbild des stereoskopischen Endoskopbilds in dem Überlagerungsbild eine Raumwirkung, also ein Tiefeneindruck, in der bestimmten Raumrichtung erzeugt wird. Hierdurch kann vorteilhaft eine verbesserte Anpassung zwischen dem Endoskopbild beziehungsweise dessen stereoskopischer Tiefenlage und dem 3D-Datensatz oder aus diesem erzeugten Anteilen des Überlagerungsbildes erreicht werden. Dadurch kann dem Betrachter oder Anwender vorteilhaft ein verbesserter, insbesondere realistischerer Eindruck vermittelt werden.

Es sind hier mehrere Ausführungsformen der vorliegenden Erfindung beschrieben, in welchen ein Endoskop verwendet wird. Jeweilige Ausgestaltungsdetails oder Erläuterungen, welche im Zusammenhang mit einer oder mehreren dieser Ausführungsformen beschrieben sind, sollen dabei in sinngemäß entsprechender Weise auch für die anderen Ausführungsformen gelten.

In weiterer vorteilhafter Ausgestaltung der vorliegenden Erfindung wird automatisch ein Pfad bestimmt und als das Signal ausgegeben, entlang welchem das Instrument zu führen ist, um unter Berücksichtigung der, insbesondere richtungsabhängigen, Unsicherheit der 2D/3D-Registrierung ein vorgegebenes Ziel zu erreichen.

Der Pfad kann dabei nicht nur eine Reihe von Wegpunkten oder Positionen, sondern ebenfalls jeweilige Orientierungsinformationen, also an den Punkten des Pfades oder entlang des Pfades einzunehmende oder einzustellende Orientierungen oder Posen des Instruments umfassen oder angeben.

Das vorgegebene Ziel kann ein Zielort oder eine Zielpose für das Instrument, eine bestimmte Stelle des Zielobjekts, eine Probenentnahme beispielsweise aus einem bestimmten Gewebe, Gewebeabschnitt oder Teil des Zielobjekts, eine Einwirkung auf ein bestimmtes Gewebe, einen bestimmten Gewebeabschnitt oder einen bestimmten Teil des Zielobjekts und/oder dergleichen mehr sein, umfassen oder angeben. Dazu kann insbesondere eine aus dem 3D-Datensatz und/oder aus dem 2D-Bild abgeleitete und/oder vorgegebene Struktur des Zielobjekts berücksichtigt werden, welche mögliche Pfade und/oder Ausrichtungen des Instruments einschränken oder limitieren kann.

Der Pfad kann bevorzugt beispielsweise so bestimmt werden, dass sich entlang des Pfades das Instrument möglichst wenig in der bestimmten Raumrichtung der größten Unsicherheit und/oder mit möglichst geringer Abweichung zwischen der Ausrichtung des Instruments und der bestimmten Raumrichtung bewegt oder bewegen kann. Dadurch kann insgesamt eine Positioniergenauigkeit des Instruments in Bezug auf das Zielobjekt beziehungsweise das vorgegebene Ziel verbessert oder maximiert und dementsprechend das vorgegebene Ziel mit größerer Genauigkeit oder Zuverlässigkeit und gegebenenfalls verringerter Belastung des Zielobjekts erreicht werden. Typischerweise hat beispielsweise das Instrument einen begrenzten Wirkungsbereich. Aufgrund der Unsicherheit der 2D/3D Registrierung kann dann beispielsweise in der bestimmten Raumrichtung ein zusätzlicher Wegpunkt oder Wirkungs-, also Einsatzort, des Instruments als Teil des Pfades geplant oder vorgesehen werden, um trotz der Registrierungsunsicherheit sicherzustellen, dass das vorgegebene Ziel, beispielsweise eine Läsion des Zielobjekts, von dem Wirkungsbereich des Instruments erfasst oder überdeckt wird. Die Wirkung des Instruments kann dabei beispielsweise ein Abbilden des vorgegebenen Ziels, gegebenenfalls mit einer vorgegebenen minimalen Bildschärfe, ebenso wie ein Beeinflussen des Zielobjekts beziehungsweise des vorgegebenen Ziels, beispielsweise ein Entnehmen einer Probe und/oder eine Ablation, sein oder umfassen.

In vorteilhafter Weiterbildung der vorliegenden Erfindung wird zum Bestimmen des Pfades eine Vorzugsrichtung einer Wirkung des Instruments berücksichtigt. Mit anderen Worten wird also eine räumliche Anisotropie der Wirkung oder eines Wirkungsbereiches des Instruments berücksichtigt. Es kann beispielsweise möglich sein, dass das Instrument eine Biopsienadel zur Probenentnahme ist, mittels welcher entlang des Pfades, also in einer Bewegungsrichtung der Biopsienadel, nicht aber in hierzu seitlichen Richtungen mehrere Proben entnommen werden können.

Um sicherzustellen, dass tatsächlich eine Probe aus dem jeweils vorgegebenen Ziel oder Zielbereich entnommen wird, kann es dann beispielsweise besonders vorteilhaft vorgesehen sein, dass der Pfad so geplant oder bestimmt wird, dass sich das Instrument bei dessen Führung entlang des bestimmten Pfades im Bereich des vorgegebenen Ziels je nach Anwendungsfall, beispielsweise je nach Form des vorgegebenen Zielbereiches, entlang oder beispielsweise senkrecht zu der bestimmten Raumrichtung der größten Unsicherheit der 2D/3D-Registrierung bewegt. Ebenso kann durch die Berücksichtigung der Vorzugsrichtung des Instruments beziehungsweise der Wirkung des Instruments beispielsweise eine maximale Überdeckung zwischen dem Wirkungsbereich des Instruments und dem vorgegebenen Ziel unter Berücksichtigung der Registrierungs- und Visualisierungsungenauigkeit oder -unsicherheit durch entsprechendes Planen oder Bestimmen des Pfades erreicht werden. Beispielsweise kann eine Ablationszone bei einer Nadelablation nicht kreis- oder kugelförmig, sondern elliptisch entlang einer Achse einer entsprechenden verwendeten Ablationsnadel ausgedehnt sein. Soll dann beispielsweise ein Tumorgewebe entfernt werden, so kann durch die vorliegende Erfindung mit verbesserter Wahrscheinlichkeit oder Zuverlässigkeit sichergestellt werden, dass das Tumorgewebe sich tatsächlich vollständig innerhalb eines Volumens oder Wirkungsbereiches der Ablation, also innerhalb der Ablationszone, befindet und dabei umgebendes Gewebe möglichst nicht oder möglichst wenig in Mitleidenschaft gezogen wird.

Es kann durch die vorliegende Erfindung also vorteilhaft ein verbesserter Pfad für ein interventionelles oder chirurgisches Instrument bestimmt werden, dessen Wirkung auf ein Zielobjekt, beispielsweise ein Körpergewebe, eine geometrische Vorzugsrichtung oder räumliche Anisotropie aufweist. Dies wird erreicht durch Berücksichtigung dieser Vorzugsrichtung oder Anisotropie und der räumlich anisotropen Unsicherheit oder Ungenauigkeit der 2D/3D-Registrierung und damit auch des Überlagerungsbildes.

In weiterer vorteilhafter Ausgestaltung der vorliegenden Erfindung wird das Instrument selbst in dem Überlagerungsbild dargestellt, beispielsweise als Bild oder als Modell. Als das Signal oder als Teil des Signals oder durch das Signal wird die Darstellung des Instruments und/oder dessen Einfluss- oder Wirkungsbereiches in dem Überlagerungsbild dann in Abhängigkeit von der jeweiligen lokalen Unsicherheit der 2D/3D-Registrierung räumlich anisotrop verbreitert. Mit anderen Worten kann also die Darstellung des Instruments und/oder dessen Wirkungsbereiches gemäß der Unsicherheit der 2D/3D-Registrierung und somit einer Unsicherheit der Visualisierung oder Darstellung des Überlagerungsbild verzerrt werden. Da der jeweilige Anwender typischerweise mit dem Instrument vertraut ist, kann ihm auf diese Weise die Art, Größe und Richtung der Unsicherheit oder eines entsprechenden Visualisierungsfehler besonders einfach, intuitiv erfassbar und ablenkungsarm angezeigt werden. Weist das Instrument real beispielsweise eine zylindrische oder stabförmige Form oder Gestalt auf, so kann diese in dem Überlagerungsbild je nach aktueller Ausrichtung des Instruments in Bezug auf die bestimmte Raumrichtung beispielsweise elliptisch verbreitert werden. Entsprechend kann die Darstellung des Wirkungsbereiches angepasst werden.

Ebenso kann das anisotrope Verbreitern der Darstellung bedeuten, dass die Darstellung des Instruments in dem Überlagerungsbild von einer entsprechenden Verbreiterungs- oder Unsicherheitszone, also einem entsprechend gekennzeichneten Raumbereich, umgeben wird oder ist. Es kann dann beispielsweise ein Pfad für das Instrument automatisch oder durch den jeweiligen Anwender geplant oder verfolgt werden, welcher das Instrument so führt, dass die gesamte verbreitete Darstellung des Instruments beziehungsweise seines Wirkungsbereiches durch das vorgegebene Ziel, beispielsweise eine Läsion oder dergleichen, hindurch verläuft, diese also vollständig erfasst. Auf diese Weise kann vorteilhaft sichergestellt werden, dass trotz der Registrierungsunsicherheit alle möglichen Instrumentenpfade innerhalb eines durch die verbreitete Darstellung angegebenen oder angezeigten Bereiches tatsächlich durch das vorgegebene Ziel, beispielsweise die Läsion oder einen Tumor oder dergleichen, verlaufen oder diesen treffen. Insbesondere wenn der Wirkungsbereich des Instruments kleiner als die verbreitete Darstellung ist, können dementsprechend beispielsweise mehrere Wirkungs- oder Einsatzorte, also beispielsweise mehrere Probenentnahmepunkte, oder mehrere nebeneinander verlaufende Pfade durch das vorgegebene Ziel oder im Bereich des vorgegebenen Ziels geplant oder verfolgt werden, um das vorgegebene Ziel mit der gesamten verbreiterten Darstellung und/oder mit dem Wirkungsbereich des Instruments in Überdeckung zu bringen.

Handelt es sich bei dem Instrument beispielsweise um eine Nadel und ist die Unsicherheit der 2D/3D-Registrierung in x- und y-Richtung relativ klein, in z-Richtung jedoch relativ groß, so würde die Nadel bei Ausrichtung in oder entlang der z-Richtung beispielsweise als Linie dargestellt und bei zunehmender Abweichung von dieser Ausrichtung immer weiter verbreitert dargestellt werden. Eine Ausrichtung der Nadel in oder entlang der z-Richtung bedeutet dabei, dass eine Längs- oder Haupterstreckungsrichtung der Nadel in z-Richtung verläuft.

In weiterer vorteilhafter Ausgestaltung der vorliegenden Erfindung wird in Abhängigkeit von der richtungsabhängigen Unsicherheit der 2D/3D-Registrierung, insbesondere unter Berücksichtigung einer Vorzugsrichtung einer Wirkung des Instruments, automatisch wenigstens eine Einsatzstelle des Instruments bestimmt und als das Signal ein entsprechender Hinweis, also entsprechende Positionsdaten, ein entsprechender Vorschlag oder eine entsprechende Kennzeichnung, ausgegeben. Die Einsatzstelle ist in diesem Sinne also ein Ort oder eine Position, insbesondere einschließlich einer zugehörigen Orientierung des Instruments, an welcher das Instrument seine bestimmungsgemäße Wirkung entfalten soll und/oder zum Entfalten seiner Wirkung positioniert werden soll. Handelt es sich bei dem Instrument beispielsweise um eine Biopsie- oder Ablationsnadel, kann die Einsatzstelle beispielsweise eine Stelle oder einen Teil des Zielobjekts angeben, an welcher beziehungsweise aus welchem mittels der Biopsienadel eine Probe entnommen werden soll oder an welcher beziehungsweise an welchem mittels der Ablationsnadel Material oder Gewebe ablatiert werden soll. Es kann also automatisch in Abhängigkeit von der richtungsabhängigen Registrierungsunsicherheit unter Berücksichtigung der Wirkungsrichtung und der Anisotropie des Wirkungsbereiches des Instruments eine Empfehlung für wenigstens eine Einsatzstelle, also einen Wirkungs- oder Anwendungsort, für das Instrument bestimmt und ausgegeben werden. Auf diese Weise kann automatisch besonders zuverlässig sichergestellt werden, dass die Wirkung des Instruments tatsächlich ein vorgegebenes Ziel trotz der Registrierungsunsicherheit erreicht. Ohne den entsprechenden Hinweis kann es ansonsten aufgrund der Registrierungsunsicherheit und eines gegebenenfalls resultierenden Visualisierungsfehlers für einen Anwender nur schwierig abschätzbar sein, ob und gegebenenfalls mit welcher Wahrscheinlichkeit das vorgegebene Ziel bei einer jeweils aktuellen Positionierung und Ausrichtung des Instruments tatsächlich erreicht wird.

In weiterer vorteilhafter Ausgestaltung der vorliegenden Erfindung wird automatisch eine jeweils aktuelle Ausrichtung des Instruments erfasst, insbesondere nachverfolgt. In Abhängigkeit von der erfassten Ausrichtung des Instruments und der Unsicherheit der 2D/3D-Registrierung wird dann automatisch eine Wahrscheinlichkeit dafür berechnet, dass mittels des Instruments in der erfassten Ausrichtung ein vorgegebenes Ziel erreicht wird. Die berechnete Wahrscheinlichkeit wird dann als das Signal oder als Teil des Signals ausgegeben. Es kann also beispielsweise mittels einer statistischen Analyse, etwa einer Monte-Carlo-Simulation oder dergleichen, berechnet werden, mit welcher Wahrscheinlichkeit ein bestimmter Pfad des Instruments oder eine Kombination mehrerer Pfade des Instruments dazu führen wird, dass das vorgegebene Ziel, beispielsweise eine Läsion oder ein Tumor oder eine Probenentnahme aus einem bestimmten Gewebebereich, tatsächlich erreicht oder erfüllt wird. Diese Wahrscheinlichkeit wird dann dem jeweiligen Anwender angezeigt, welcher daraufhin den Pfad oder die Ausrichtung des Instruments anpassen kann und/oder eine verbesserte, insbesondere genauere, Registrierung durchführen kann, bis eine im jeweiligen Einzelfall ausreichende Wahrscheinlichkeit für das Erreichen des Ziels erreicht oder gegeben ist. Auf diese Weise kann statistisch objektiv ein Erfolg einer jeweiligen Anwendung verbessert oder überprüfbar gemacht, also nachvollziehbar prognostiziert werden. Ergibt beispielsweise eine Untersuchung einer entnommenen Probe ein negatives Ergebnis, so kann dieses anhand der bei der Probenentnahme durch den verwendeten Pfad des Instruments gegebenen Wahrscheinlichkeit dafür, dass die Probe aus dem vorgegebenen Zielgewebe entnommen wurde, eingestuft oder bewertet werden. Wird durch Verwendung der vorliegenden Erfindung überprüfbar sichergestellt, dass die Wahrscheinlichkeit für das Erreichen des Ziels bei 100% oder nahe bei 100% liegt oder lag, kann so gegebenenfalls eine weitere Probenentnahme vermieden werden.

Besonders vorteilhaft kann dabei zudem eine Unsicherheit oder Ungenauigkeit oder Toleranz bei einer Führung oder Positionierung und/oder bei einem Nachverfolgen des Instruments als statistisch unabhängige Größe zu der Unsicherheit der 2D/3D-Registrierung addiert, also mit dieser kombiniert werden. Auf diese Weise kann die Wahrscheinlichkeit für das Erreichen des vorgegebenen Ziels noch genauer und zuverlässiger berechnet werden. In entsprechender Weise können einige oder alle dieser Unsicherheiten oder Wahrscheinlichkeiten ebenso beispielsweise bei dem beschriebenen Anpassen der Darstellung des Instruments, beispielsweise durch eine entsprechend größere Verbreiterung der Darstellung, berücksichtigt werden. Auf diese Weise kann dem jeweiligen Anwender beispielsweise vermittelt werden, wie weit und/oder mit welcher Wahrscheinlichkeit ein tatsächlicher Pfad oder eine tatsächliche Position des Instruments von einem geplanten Pfad oder einer geplanten Position abweichen kann.

Unabhängig von der jeweils verwendeten Ausführungsformen der vorliegenden Erfindung kann es vorgesehen sein, dass mehrere 2D-Bilder nacheinander erfasst werden, beispielsweise in Form einer Fluoroskopie. Es kann dann automatisch gemäß einem jeweils aktuellen 2D-Bild und dessen jeweiliger Aufnahmerichtung automatisch eine Aktualisierung durchgeführt, also die Raumrichtung der größten Unsicherheit der 2D/3D-Registrierung jeweils erneut bestimmt oder aktualisiert, die 2D/3D-Registrierung jeweils erneut durchgeführt oder aktualisiert und/oder ein entsprechend aktualisiertes Signal erzeugt und ausgegeben werden.

Ein weiterer Aspekt der vorliegenden Erfindung ist ein Computerprogrammprodukt, welches ein Computerprogramm umfasst. Dieses Computerprogramm kodiert oder repräsentiert dabei ein erfindungsgemäßes Verfahren, also die Verfahrensschritte zumindest einer Ausführungsform des erfindungsgemäßen Verfahrens. Das erfindungsgemäße Computerprogrammprodukt beziehungsweise das Computerprogramm ist dabei ausgebildet und eingerichtet zum Laden in eine Speichereinrichtung einer Datenverarbeitungseinrichtung, insbesondere eines bildgebenden Systems, und zum Ausführen des Verfahrens, wenn das Computerprogramm mittels der Datenverarbeitungseinrichtung ausgeführt wird. Die Datenverarbeitungseinrichtung kann dazu eine entsprechende mit der Speichereinrichtung verbundene Prozessoreinrichtung, also beispielsweise wenigstens einen Mikrochip, oder Mikrocontroller, umfassen. Das erfindungsgemäße Computerprogrammprodukt kann also Programmmittel umfassen, um das erfindungsgemäße Verfahren auszuführen. Diese Programmmittel können dabei weitere, hier nicht explizit genannte Komponenten, beispielsweise entsprechende Steuerbefehle, Registerverweise und/oder dergleichen mehr, umfassen.

Ein weiterer Aspekt der vorliegenden Erfindung ist ein, insbesondere in elektronischer und/oder elektronisch lesbarer und/oder computerlesbarer, Datenträger oder Datenspeicher, insbesondere für ein bildgebendes System. Auf dem erfindungsgemäßen Datenträger ist dabei ein mittels einer Datenverarbeitungseinrichtung, insbesondere automatisch, ausführbarer Programmcode gespeichert, welcher ein Computerprogramm umfasst, welches zumindest eine Ausführungsform des erfindungsgemäßen Verfahrens, also entsprechende Verfahrensschritte kodiert oder repräsentiert, um bei einer Ausführung des Computerprogramms oder des Programmcodes durch die Datenverarbeitungseinrichtung eine Ausführung des Verfahrens, also der entsprechenden Verfahrensschritte, zu bewirken.

Auf dem erfindungsgemäßen Datenträger kann also insbesondere zumindest eine Ausführungsform des erfindungsgemäßen Computerprogrammprodukts beziehungsweise des von diesem umfassten Computerprogramms gespeichert sein.

Ein weiterer Aspekt der vorliegenden Erfindung ist ein bildgebendes System. Das erfindungsgemäße bildgebende System weist eine Erfassungseinrichtung zum Erfassen eines ein Zielobjekt abbildenden 3D-Datensatzes und wenigstens eines 2D-Bildes des Zielobjekts auf. Der 3D-Datensatz kann dabei beispielsweise mittels eines anderen Systems bereits zu einem früheren Zeitpunkt aufgenommen worden sein. In diesem Fall kann das Erfassen des 3D-Datensatzes etwa dessen Abrufen aus einem elektronischen oder computer-lesbaren Datenspeicher oder dergleichen bedeuten. Ebenso kann die Erfassungseinrichtung selbst zum Aufnehmen, also Messen des 3D-Datensatzes ausgebildet und eingerichtet sein. Weiter weist das erfindungsgemäße bildgebende System eine Datenverarbeitungseinrichtung auf, welche eine Prozessoreinrichtung und einen mit dieser verbundenen erfindungsgemäßen Datenträger umfasst. Weiter weist das erfindungsgemäße bildgebende System eine Ausgabeeinrichtung zum Ausgeben eines automatisch erzeugten Signals auf. Die Ausgabeeinrichtung kann dabei beispielsweise eine Schnittstelle der Datenverarbeitungseinrichtung, ein Programmmodul des Computerprogramms oder Programmcodes und/oder eine Anzeigeeinrichtung wie etwa ein Bildschirm oder ein HMD sein oder umfassen. Das erfindungsgemäße bildgebende System ist also insbesondere zum Durchführen oder Ausführen des erfindungsgemäßen Verfahrens ausgebildet und eingerichtet. Dementsprechend kann erfindungsgemäße bildgebende System also einige oder alle der im Zusammenhang mit den übrigen Aspekten der vorliegenden Erfindung, also im Zusammenhang mit dem erfindungsgemäßen Verfahren, dem erfindungsgemäßen Computerprogrammprodukt und dem erfindungsgemäßen Datenträger, genannten Eigenschaften und/oder Komponenten oder Bauteile aufweisen.

Das erfindungsgemäße bildgebende System kann dabei als kompaktes, also integriertes oder beispielsweise in einem Gehäuse angeordnetes, System oder Gerät ausgebildet sein. Ebenso können zumindest einige Komponenten oder Bauteile des bildgebenden Systems, insbesondere also die Erfassungseinrichtung und die Datenverarbeitungseinrichtung, an unterschiedlichen, räumlich verteilten oder räumlich voneinander beabstandeten, Orten angeordnet sein. Die Datenverarbeitungseinrichtung des bildgebenden Systems kann sich also - beispielsweise als Rechenzentrum oder als Teil eines Rechenzentrums - etwa in einem anderen Raum als die Erfassungseinrichtung befinden. Die Datenverarbeitungseinrichtung kann dabei "on-premise" angeordnet sein. Das kann beispielsweise bedeuten, dass die Datenverarbeitungseinrichtung und die Erfassungseinrichtung, insbesondere alle Komponenten oder Bauteile des erfindungsgemäßen bildgebenden Systems, an demselben Unternehmensstandort, beispielsweise auf demselben Firmen-, Fabrik- oder Krankenhausgelände, angeordnet sein können. Ebenso kann es sich bei der Datenverarbeitungseinrichtung aber beispielsweise um eine entfernt angeordnete Servereinrichtung, beispielsweise einen Cloud- oder Remoteserver, handeln. Dieser kann dann in einem Rechenzentrum angeordnet sein und beispielsweise über ein WAN-Netzwerk (englisch: "Wide Area Network"), beispielsweise das Internet, mit der Erfassungseinrichtung des bildgebenden Systems verbunden sein.

Es sei an dieser Stelle betont, dass das erfindungsgemäße Verfahren zwar beispielsweise während oder in Vorbereitung eines interventionellen oder chirurgischen Eingriffs ausgeführt werden kann, selbst jedoch keinerlei chirurgische Schritte umfasst oder auch nur voraussetzt. Vielmehr kann das erfindungsgemäße Verfahren als Verfahren zum Betreiben eines bildgebenden Systems oder einer Datenverarbeitungseinrichtung aufgefasst werden, bei welchem empfangene Daten verarbeitet und Ausgangsdaten in Form des Signals erzeugt und ausgegeben werden. Dies ist vollständig losgelöst und unabhängig von einer konkreten interventionellen oder chirurgischen Anwendung. Ebenso dient das erfindungsgemäße Verfahren nicht zum automatischen Stellen einer Diagnose. Sofern überhaupt möglich sind entsprechende, einen chirurgischen und/oder diagnostischen Schritt umfassende Ausführungsformen des erfindungsgemäßen Verfahrens vorliegend also nicht mitbeansprucht.

Die bisher und im Folgenden angegebenen Eigenschaften und Weiterbildungen des erfindungsgemäßen Verfahrens, des erfindungsgemäßen Computerprogramms, des erfindungsgemäßen Datenträgers und des erfindungsgemäßen bildgebenden System sowie die entsprechenden Vorteile sind jeweils sinngemäß wechselseitig zwischen diesen Aspekten der vorliegenden Erfindung übertragbar. Es gehören also zu der Erfindung auch solche Weiterbildungen des erfindungsgemäßen Verfahrens, des erfindungsgemäßen Computerprogramms, des erfindungsgemäßen Datenträgers und des erfindungsgemäßen bildgebenden System, welche Ausgestaltungen aufweisen, die hier zur Vermeidung unnötiger Redundanz nicht explizit in der jeweiligen Kombination oder für jeden Aspekt der Erfindung separat beschrieben sind.

Weitere Merkmale, Einzelheiten und Vorteile der vorliegenden Erfindung ergeben sich aus der nachfolgenden Beschreibung bevorzugter Ausführungsbeispiele sowie anhand der Zeichnungen. Dabei zeigen:
- FIG 1: eine schematische Darstellung eines bildgebenden System, und
- FIG 2: ein beispielhaftes schematisches Programmschema für ein Verfahren zum Unterstützen eines Anwenders des bildgebenden System aus FIG 1.

Bei den im Folgenden erläuterten Ausführungsbeispielen handelt es sich um bevorzugte Ausführungsformen der Erfindung. Bei den Ausführungsbeispielen stellen die beschriebenen Komponenten der Ausführungsformen jeweils einzelne, unabhängig voneinander zu betrachtende Merkmale der Erfindung dar, welche die Erfindung jeweils auch unabhängig voneinander weiterbilden und damit auch einzeln oder in einer anderen als der gezeigten oder beschriebenen Kombination als Bestandteil der Erfindung anzusehen sind. Des Weiteren sind die beschriebenen Ausführungsformen auch durch weitere der bereits beschriebenen Merkmale der Erfindung ergänzbar.

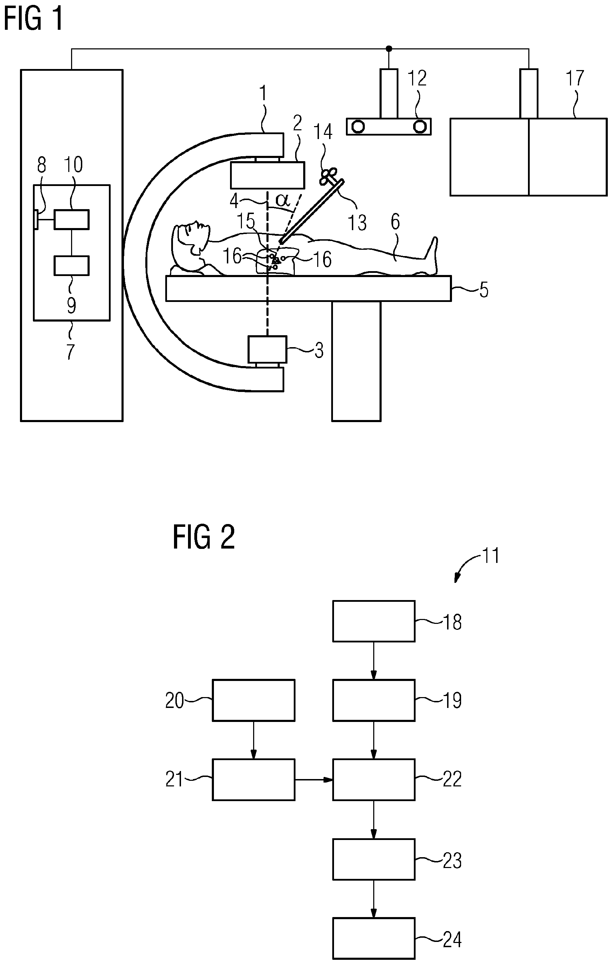

FIG 1 zeigt eine schematische Darstellung eines bildgebenden Systems 1, welches vorliegend als C-Bogens-Röntgengerät ausgebildet ist. Das bildgebende System 1 weist vorliegend eine Strahlenquelle 3 und einen Detektor 2 zum Detektieren von mittels der Strahlenquelle 3 ausgesendeter Röntgenstrahlung auf. Mittels der Röntgenstrahlung, also mittels des bildgebenden Systems 1, kann vorliegend entlang oder in einer Aufnahmerichtung 4 ein Zielobjekt abgebildet werden. Vorliegend wird als Zielobjekt ein auf einem Patientenlager 5 befindlicher Patient 6 zumindest bereichsweise mittels des bildgebenden Systems 1 abgebildet. Weiter weist das bildgebende System 1 eine Datenverarbeitungseinrichtung 7 zum Verarbeiten von dem Detektor 2 bereitgestellten Sensor- oder Bilddaten sowie gegebenenfalls weiterer, über eine Schnittstelle 8 der Datenverarbeitungseinrichtung 7 empfangener oder erfasster Daten auf. Die Datenverarbeitungseinrichtung 7 weist weiter eine Speichereinrichtung 9 sowie eine mit der Speichereinrichtung 9 und mit der Schnittstelle 8 verbundene Prozessoreinrichtung 10 auf. Auf oder in der Speichereinrichtung 9 ist vorliegend ein Computerprogramm, also ein Programmcode, gespeichert, welcher ein Verfahren zum Unterstützen eines Anwenders kodiert oder repräsentiert. Zum Ausführen dieses Verfahrens kann dieses Computerprogramm beziehungsweise dieser Programmcode mittels der Prozessoreinrichtung 10 ausgeführt werden.

FIG 2 zeigt beispielhaft und ausschnittweise ein Programmschema 11 mit schematisch angedeuteten Programmmodulen 18 bis 24 für ein derartiges Verfahren zum Unterstützen eines Anwenders des bildgebenden Systems 1.

Vorliegend umfasst das bildgebende System 1 weiter eine Nachverfolgungseinrichtung 12 (englisch: "tracking system"), welche hier beispielsweise als Stereokamera und/oder für ein elektromagnetisches Nachverfolgen ausgebildet ist. Mittels der Nachverfolgungseinrichtung 12 kann ein Instrument, wie etwa ein hier dargestelltes Laparoskop 13, nachverfolgt werden. Um dieses Nachverfolgen, also ein kontinuierliches oder regelmäßiges Erfassen des Laparoskops 13 zu ermöglichen, weist dieses vorliegend einen entsprechenden Marker 14 auf. Mittels des Laparoskops 13 kann der Patient 6 in einer Blick- oder Erfassungsrichtung 15 des Laparoskops 13 optisch abgebildet werden.

Vorliegend sind in dem Patienten 6 mehrere Patientenmarker 16 angeordnet, welche ebenfalls mittels der Nachverfolgungseinrichtung 12 zum Bestimmen und Nachverfolgen einer Pose des Patienten 6 erfasst werden können.

Weiter umfasst das bildgebende System 1 vorliegend eine Anzeigeeinrichtung 17, welche hier beispielhaft als Bildschirm oder Monitor ausgebildet ist.

Eine Verwendung des bildgebenden Systems 1 zum Unterstützen des Anwenders und das in FIG 2 dargestellte Programmschema 11 werden im Folgenden unter Bezugnahme auf FIG 1 und FIG 2 näher erläutert.

In medizinischen oder chirurgischen Anwendungen wird heutzutage oftmals eine augmentierte Realität (englisch: augmented reality") verwendet. Vorliegend wird dazu mittels des Programmmoduls 18 ein 3D-Datensatz, beispielsweise ein präoperativer oder präinterventioneller 3D-Volumenscan des Patienten 6, erfasst. Bei der Intervention wird dann mittels des Programmmoduls 18 und dem bildgebenden System 1 wenigstens ein 2D-Bild des Patienten 6 erfasst. Zudem mittels des Programmmoduls 18 ein Endoskopbild und oder Endoskopvideo des Patienten 6 erfasst, welches vorliegend mittels des Laparoskops 13 aufgenommen wird. Ziel ist es nun zunächst aus diesen verschiedenen Daten ein Überlagerungsbild, also ein AR-Bild, zu erzeugen.

Beispielsweise in der chirurgischen augmentierten Realität müssen dabei dem Endoskopbild oder Endoskopvideo, hier also einem Laparoskopbild oder Laparoskopvideo, überlagerte Strukturen oder Einblendungen möglichst präzise zu einer realen Anatomie des Patienten 6 registriert sein. Ebenso ist eine möglichst präzise Registrierung zu der realen Anatomie des Patienten 6 beispielsweise bei einer navigierten Führung von Nadeln, zum Beispiel basierend auf einer Laserführung oder einer optischen Navigation notwendig. Oftmals werden dazu Nadelpfade, also Pfade entlang welchen die Nadeln oder ein entsprechendes Instrument bewegt oder geführt werden sollen, auf Basis einer 2D/3D-Registrierung oder einer 2D/2D/3D-Registrierung von einer oder zwei 2D-Röntgenprojektionen, also 2D-Röntgenbildern, zu dem 3D-Volumenscan oder einem entsprechenden 3D-Datensatz berechnet. Ein weiteres Beispiel ist eine Nadelführung mithilfe eines 2D- oder 3D-Ultraschalls, bei der ein Algorithmus eine 2D/3D-Registrierung zu einem 3D-Ultraschallscan oder zu einem 3D-CT- oder -MR-Scan errechnet. Dank der 2D/3D- oder 2D/2D/3D-Registrierung muss während der Nadelintervention dann kein erneuter 3D-Scan durchgeführt werden oder es kann über weitere während der Intervention aufgenommene Röntgen- oder Projektionsbilder eine schnelle Bewegungsaktualisierung nach einem intraoperativen 3D-Scan erzielt werden.

Die beschriebenen Registrierungen sind in vielen Fällen allerdings nicht in allen Raumrichtungen gleich präzise. Oftmals gibt es etwa den Fall, dass die Registrierung in zwei zueinander orthogonalen Raumrichtungen, welche hier ohne Einschränkung der Allgemeinheit als x-Richtung und y-Richtung bezeichnet werden, mit relativ hoher Präzision erfolgt, während eine dritte Raumrichtung, also eine z-Richtung, nur sehr ungenau registriert werden kann. Konkret tritt dieser Fall beispielsweise auf, wenn während einer Operation oder Intervention mittels des bildgebenden Systems 1 ein 2D-Röntgenbild entlang der z-Richtung oder z-Achse, welche hier dann der Aufnahmerichtung 4 entspricht oder entsprechen kann, aufgenommen wird. Zu diesem 2D-Röntgenbild soll dann mittels einer 2D/3D-Registrierung ein zuvor aufgenommener oder erfasster also bereitgestellter, 3D-Datensatz registriert werden.

Vorliegend wird diese 2D/3D-Registrierung oder 2D/2D/3D-Registrierung mittels eines Programmmoduls 19 durchgeführt. Die 2D/3D-Registrierung wird anschließend verwendet, um den 3D-Datensatz oder eine daraus abgeleitete Darstellung wie beispielsweise ein segmentiertes Mesh mittels oder in Form einer AR-Darstellung, also mittels in Form augmentierter Realität, dem Endoskopbild zu überlagern. Zusätzlich oder alternativ wird die 2D/3D-Registrierung als Basis für eine Pfadplanung einer Nadel, beispielsweise einer Biopsienadel oder Ablationsnadel, oder eines anderen Instruments verwendet.

Dazu werden vorliegend durch das Programmodul 20 das Laparoskop 13 anhand des Markers 14 und der Patient 6 anhand der Patientenmarker 16, also eine jeweilige Pose des Laparoskops 13 und des Patienten 6, erfasst und nachverfolgt. Somit ist also die Erfassungsrichtung 15 des Laparoskops 13 bekannt, steht also für eine Datenverarbeitung zur Verfügung. Aus entsprechenden Steuer- oder Betriebsparametern des bildgebenden Systems 1 ist zudem die Aufnahmerichtung 4 bekannt, also für die Datenverarbeitung verfügbar. Durch das Programmodul 21 wird die Erfassungsrichtung 15 relativ zu der Aufnahmerichtung 4, also ein Winkel α zwischen der Aufnahmerichtung 4 und der Erfassungsrichtung 15, bestimmt.

Problematisch ist, dass die 2D/3D-Registrierung im Allgemeinen beispielsweise in x-Richtung und in y-Richtung relativ präzise, in z-Richtung, also entlang der Aufnahmerichtung 4, jedoch deutlich unpräziser ist. Diese Ungenauigkeit oder Unsicherheit entlang einer Raumachse oder Raumrichtung kann sich bei dem Erzeugen des AR- oder Überlagerungsbildes, also bei dem Überlagern des 3D-Datensatzes oder daraus erzeugter Überlagerungsstrukturen auf dem Endoskopvideo, für einen Betrachter oder den Anwender sehr störend und je nach Blickrichtung des Endoskops 13, also je nach relativer Ausrichtung der Erfassungsrichtung 15 unterschiedlich stark auswirken oder bemerkbar machen. Eine unpräzise Überlagerung, welche dem Anwender, beispielsweise einem Chirurgen, nicht bewusst ist, birgt die Gefahr einer Fehlentscheidung, wobei im schlimmsten Fall beispielsweise an einer falschen Stelle des Patienten 6 oder eines bestimmten Organs ein Schnitt gesetzt werden kann. Beispielsweise bei Nadelbiospien möchte man hingegen sicher sein, eine mittels bis 3D-Datensatzes, also einer 3D-Bildgebung, gefundene Läsion auch tatsächlich entlang des Nadelpfades zu treffen. Es kann dabei beispielsweise die Möglichkeit bestehen, entlang des Nadelpfades mehrere Biopsien, also Proben, zu entnehmen, wobei seitlich von dem Nadelpfad abweichende Probenorte aber nicht untersucht oder erreicht werden können.

In einem weiteren Beispiel ist eine Ablationszone bei einer Nadelablation nicht kreisförmig, sondern entlang einer Nadelachse ausgedehnter als in den anderen Richtungen. Ein Tumor soll dabei aber komplett innerhalb eines Volumens der Ablationszone liegen.

Allgemein soll bei einer Planung eines Anwendungspfades eines interventionellen oder chirurgischen Instruments, dessen Wirkung auf ein Körpergewebe eine geometrische Vorzugsrichtung oder Anisotropie aufweist, eine räumliche Anisotropie der Genauigkeit oder Sicherheit beziehungsweise Unsicherheit der 2D/3D-Registrierung berücksichtigt werden.

Bisher gab es für den Anwender keinerlei Hilfe oder Indikation, in welcher Richtung die 2D/3D-Registrierung und damit die Überlagerung, also das Überlagerungs- oder AR-Bild genau oder ungenau registriert oder visualisiert ist. Ebenso findet die räumlich anisotrope Präzision oder Sicherheit der 2D/3D-Registrierung bisher keine Berücksichtigung bei der Planung von Nadelfaden.

Ein Ausweg kann darin bestehen, durch Verwendung mehrerer Röntgenprojektionsrichtungen, also durch mehrere 2D-Röntgenbilder aus oder mit verschiedenen Angulationen, eine Verwendung eines Biplan-Röntgensystems oder einer Nutzung von 3D-Röntgenbildern auch während der jeweiligen Intervention, eine Registrierungsgenauigkeit oder Registrierungssicherheit in allen Raumrichtungen zu verbessern oder sicherzustellen. Dadurch können sich jedoch Nachteile in einem chirurgischen Workflow, eine höhere Dosis oder Belastung für den Patienten 6 und gegebenenfalls die nachteiligen Notwendigkeit einer Verwendung eines aufwändigeren und teureren bildgebenden Systems 1 ergeben.

Im vorliegenden Beispiel ist das bildgebende System 1 als C-Bogens-Röntgengerät für eine intraoperative 3D-Bildgebung und 2D-Fluoroskopie ausgebildet. Eine optische Live-Bildgebung erfolgt mittels des Laparoskops 13. Dieses weist hier eine abgewinkelte Optik auf, sodass die Erfassungsrichtung 15 des Laparoskops 13 relativ zu einer Längserstreckungsrichtung oder einem Schaft oder Gehäuse des Laparoskops 13 abgewinkelt ist, beispielsweise um 30°. Vorliegend soll bei dem auf dem Patientenlager 5, beispielsweise auf einem OP-Tisch, liegenden Patient 6 beispielsweise eine laparoskopische Untersuchung durchgeführt werden. Beispielsweise soll ein Lebertumor reseziert werden. Der Anwender, also etwa ein Chirurg, verwendet dazu das mittels der Nachverfolgungseinrichtung 12 hinsichtlich seiner Position und Orientierung, also seiner Pose, nachverfolgte Laparoskop 13. Optional können weitere, hier nicht im Einzelnen dargestellte chirurgische Instrumente, wie beispielsweise eine Zange, ein Pointer, ein Elektrokauter und/oder dergleichen mehr, mittels der Nachverfolgungseinrichtung 12 oder mittels eines weiteren Trackingsystems erfasst und nachverfolgt werden. Ebenso kann beispielsweise eine Positions- und Lagebestimmung des Laparoskops 13 anhand von mittels des bildgebenden Systems 1 aufgenommenen Röntgen- oder Fluoroskopiebildern möglich sein.

Die Nachverfolgungseinrichtung 12 oder ein Koordinatensystem der Nachverfolgungseinrichtung 12, in welchem das Laparoskop 13 erfasst und nachverfolgt wird, ist dabei zu einem Koordinatensystem der mittels des bildgebenden Systems 1 aufgenommenen Röntgenbilder und dem 3D-Datensatz kalibriert. So kann über eine vorgegebene Koordinatentransformation berechnet werden, wie Objekte, deren 3D-Koordinaten aus den Röntgenbildern und/oder aus dem 3D-Datensatz berechnet wurden, in dem mittels des Laparoskops 13 aufgenommenen Endoskopbild oder einem entsprechenden Koordinatensystem ortsrichtig überlagert dargestellt werden können.

Das Laparoskop 13 kann dabei ein Stereo-Laparoskop sein. Ebenso kann die Anzeigeeinrichtung 17 eine stereoskopische Anzeigeeinrichtung, beispielsweise ein 3D-Monitor oder ein Stereo-Head-Mounted Display (HMD), sein.

In einem Idealfall blickt das Laparoskop 13 parallel ausgerichtet zur Röntgen-Projektionsrichtung des bildgebenden Systems 1. Das bedeutet vorliegend, dass dann also die Erfassungsrichtung 15 parallel oder antiparallel zu der Aufnahmerichtung 4 ausgerichtet ist. Eine Bildebene des Endoskopbildes ist dann entlang der Raumrichtungen x, y orientiert. In dem Überlagerungsbild können dann die aus dem 3D-Datensatz abgeleiteten oder erzeugten virtuellen Überlagerungsstrukturen dem Endoskopbild dank der relativ präzisen Registrierung in x- und y-Richtung ortsgetreu, also passgenau eingeblendet werden. Die beschriebene Registrierungsungenauigkeit oder Registrierungsunsicherheit in z-Richtung, also entlang der Aufnahmerichtung 4, macht sich dann effektiv nicht oder nicht signifikant bemerkbar.

Handelt es sich bei dem Laparoskop 13 um ein Stereo-Laparoskop, kann zusätzlich noch über eine Vergenz und/oder Parallaxe der Einblendung zwischen einem linken und einem rechten Teilbild des Stereo-Endoskopbildes ein 3D-Tiefeneindruck erzeugt werden, wobei die entsprechende Tiefenrichtung in diesem Fall ebenfalls parallel zu der Röntgen-Projektionsrichtung z, also der Aufnahmerichtung 4, ist. Die 2D/3D-Registrierung beziehungsweise die Überlagerung in dem Überlagerungsbild kann hier zwar ungenau sein, das Auge eines Betrachters oder Anwenders nimmt jedoch in der Realität relativ kleine Abweichungen oder Ungenauigkeiten in Tiefenrichtung als nicht signifikant störend war, sodass die Ungenauigkeit oder Unsicherheit in der Tiefenrichtung in diesem Fall toleriert werden kann.

In einem allgemeinen Fall ist der Winkel α zwischen der Blick- oder Erfassungsrichtung 15 des Laparoskops 13 und der Röntgen-Projektionsrichtung, also der Aufnahmerichtung 4, von Null verschieden. Dadurch ist die Bildebene des Endoskopbildes nicht mehr parallel zu den Raumrichtungen x, y. Dadurch wirkt sich die größere Registrierungsunsicherheit oder ein größerer Registrierungsfehler in z-Richtung direkt bei der 2D-Überlagerung der präoperativen Bilder aus, beispielsweise proportional zu sinus(α).

Es soll hier eine Möglichkeit für den Anwender bereitgestellt werden, die Ungenauigkeit oder Unsicherheit der 2D/3D-Registrierung und der entsprechenden Überlagerung in dem Überlagerungsbild, also einen entsprechenden Visualisierungsfehler oder eine entsprechende Visualisierungsunsicherheit oder -ungenauigkeit, in z-Richtung zu erkennen. Weiterhin soll dem Anwender eine Hilfestellung geboten werden, das Laparoskop 13 wie oben für den Idealfall beschrieben auszurichten.

Zunächst wird mittels des Programmmoduls 22 die Registrierungsunsicherheit hinsichtlich ihrer Richtung und Größe bestimmt. Dazu können an sich bekannte Verfahren verwendet werden, beispielsweise basierend auf dem Winkel a, also einer relativen Ausrichtung der Aufnahmerichtung 4 und der Erfassungsrichtung 15 zueinander, und beispielsweise vorgegebenen Erfahrungswerten und/oder Rückgabewerten eines für die 2D/3D-Registrierung und/oder für das Erzeugen des Überlagerungsbildes verwendeten Registrierungsalgorithmus.

Mittels des Programmmoduls 23 wird dann das Überlagerungsbild erzeugt.

Mittels des Programmmoduls 24 wird dann automatisch ein Signal zum Ausrichten des Laparoskops 13 in Abhängigkeit von der bestimmten Registrierungsunsicherheit erzeugt und ausgegeben.

Weicht die Blickrichtung des Laparoskops 13, also die Erfassungsrichtung 15, von der Aufnahmerichtung 4 ab, so werden die Überlagerungen in dem Überlagerungsbild grafisch angepasst oder bearbeitet, um dem Anwender den Grad der Ungenauigkeit oder Unsicherheit zu signalisieren. Dies kann zum Beispiel durch eine Unscharfzeichnung der Überlagerungsstrukturen, beispielsweise proportional zu sinus(α), geschehen, insbesondere durch eine Unscharfzeichnung nur in z-Richtung. Weiterhin möglich sind etwa ein nach und nach erfolgendes, also räumlich graduelles, Ausgrauen der Überlagerungen oder Überlagerungsstrukturen, eine Einblendung von Pfeilen und/oder anderen Symbolen in z-Richtung und/oder eine Streckung der Überlagerungsstrukturen oder Überlagerungsinhalte in z-Richtung. Bei zu großer Richtungsabweichung, wenn also der Winkel α einen vorgegebenen Schwellenwert übersteigt, können die Überlagerungsstrukturen vollständig ausgeblendet werden.

Das bildgebende System 1 beziehungsweise ein Navigationssystem kann also eine grafische Hilfestellung bieten, welche eine jeweils aktuelle Richtungsabweichung zwischen der Aufnahmerichtung 4 und der Erfassungsrichtung 15, also eine Größe des Winkels a, anzeigt. Weiterhin bietet das bildgebende System 1 beziehungsweise das Navigationssystem eine Hilfestellung, um die Ausrichtung oder das Ausrichten der Erfassungsrichtung 15 parallel oder antiparallel zu der Aufnahmerichtung 4 zu unterstützen. Dazu kann eine Fadenkreuz-Darstellung oder -navigation vorgesehen sein. Zusätzlich oder alternativ kann die Erfassungsrichtung 15 als jeweils gegeben angenommen und eine Angulation des bildgebenden Systems 1, also des C-Bogens und damit der Aufnahmerichtung 4, per Hilfestellung an den jeweiligen Anwender oder bevorzugt automatisch derart verfahren werden, dass die Aufnahmerichtung 4 parallel oder antiparallel zu der Erfassungsrichtung 15 ausgerichtet wird. Es ist vorliegend also vorgesehen, eine Abweichung zwischen der Röntgen-Projektionsrichtung des bildgebenden Systems 1, also der Aufnahmerichtung 4, und der Blickrichtung des Laparoskops 13, also der Erfassungsrichtung 15, zu messen. Es wird dann eine Hilfestellung zur parallelen Ausrichtung der Erfassungsrichtung 15 und/oder eine Hilfestellung, insbesondere eine Automatik, zur Ausrichtung der Aufnahmerichtung 4 gegeben oder verwendet. Dabei kann dem jeweiligen Betrachter oder Anwender eine Registrierung-s und/oder Visualisierungsunsicherheit durch entsprechendes Anpassen von Überlagerungsinhalten in dem Überlagerungsbild angezeigt werden.

Vorteilhaft kann sich so eine größere Präzision der Überlagerungsinhalte, also jeweiliger Überlagerungsbilder in einer medizinischen augmentierten Realitäts-Darstellung oder - Anwendung ergeben, ohne dass dafür mehrere RöntgenProjektionsrichtungen, also mehrere verschiedene Aufnahmerichtungen 4, notwendig wären. Dies wird über eine optimierte relative Ausrichtung des Laparoskops 13 erreicht. Vorteilhaft kann hierdurch ein Risiko vermindert werden, dass der jeweilige Anwender oder Chirurg aufgrund von der in einer Raumrichtung besonders unsicheren Registrierung, welche ihm nicht bewusst ist, falsche chirurgische Entscheidungen trifft. Zudem kann vorteilhaft eine engere Integration des Laparoskops 13 und des bildgebenden Systems 1, beispielsweise in Form einer automatischen Ausrichtung des C-Bogens des bildgebenden Systems 1 basierend auf einer Blick- oder Erfassungsrichtung 15 des Laparoskops 13, realisiert werden.

Das Laparoskop 13 dient hier nur als Beispiel. Ebenso kann etwa ein beliebiges anderes Endoskop oder eine Kamera oder ein Operationsmikroskop oder dergleichen verwendet werden.

Um auf das Beispiel zurückzukommen, bei dem als Instrument eine Nadel verwendet wird, kann es beispielsweise vorgesehen sein, dass bei dem Patienten 6 eine Nadelbiopsie durchgeführt werden soll, da beispielsweise anhand eines präoperativen 3D-CT- oder -MR-Scans eine verdächtige Läsion gefunden wurde. Diese kann sich beispielsweise an einer Leber oder einer Prostata des Patienten 6 befinden. Eine Nadelführung, also eine Führung oder Positionierung, also Bewegung, der Biopsienadel findet dabei navigiert statt, wofür beispielsweise eine "Laser Needle Guidance" des bildgebenden Systems 1 oder eine optische Navigation verwendet wird.

Zur Registrierung des präoperativen 3D-Scans zu einer aktuellen intraoperativen Lage eines betroffenen Organs, also beispielswese der Leber oder Prostata, wird mittels des bildgebenden Systems 1 eine 2D-Fluoroskopieaufnahme durchgeführt. Per 2D/3D-Registrierung wird der präoperative 3D-Scan dann zur aktuellen Lage des Patienten 6 registriert. Alternativ können beispielsweise zwei 2D-Fluoroskopieaufnahmen aus unterschiedlichen Angulationen aufgenommen und eine 2D/2D/3D-Registrierung durchgeführt werden. Hier kann es aus Platzgründen, also zur Vermeidung von Kollisionen, aber beispielsweise vorkommen, dass ein Winkel zwischen diesen beiden Angulationen weitaus geringer sein kann als ein optimaler Wert von 90°, was zu einer anisotropen Genauigkeit, also einer anisotropen oder richtungsabhängigen Unsicherheit, der Registrierung führt. Mit dem hier beschriebenen Verfahren wird aber auch die Verwendung von kleineren Angulationen oder Angulationswinkel als den bisher üblicherweise empfohlenen 40° ermöglicht, wenn es beispielsweise bei großen/oder schweren Patienten entsprechend wenig Platz oder Bewegungsmöglichkeiten gibt.

Bei der Durchführung der 2D/3D-oder 2D/2D/3D-Registrierung gibt es vorliegend eine Ungenauigkeit oder Unsicherheit, welche entlang der Raumrichtung z größer ist als entlang der beiden Raumrichtungen x und y.

Es wird hier nun vorgeschlagen, diese von der Richtung der Biopsienadel abhängige und typischerweise anisotrope Ungenauigkeit oder Unsicherheit bei einer Pfadplanung dem jeweiligen Anwender oder Arzt anzuzeigen, sodass er diese bei der Festlegung des Nadelpfades berücksichtigen kann. Ebenso kann die Ungenauigkeit oder Unsicherheit entsprechend bei einer automatischen Pfadplanung berücksichtigt werden. Ein entsprechendes Planungswerkzeug (Planungstool) oder Programmodul, beispielsweise das Programmodul 24, kann zusätzlich einen empfohlenen Tiefenbereich, eine Anzahl von nacheinander zu entnehmenden Biopsien oder Proben und räumliche Abstände entsprechender Entnahmeorte zueinander bestimmen. Entsprechende Werte werden unter Berücksichtigung der Registrierungsungenauigkeit oder -unsicherheit entlang einer Nadelachse, also einer Haupterstreckungsrichtung der Biopsienadel, oder entlang des Nadelpfades bestimmt.

Eine von der Unsicherheit der Registrierung beziehungsweise des Nadelpfades oder einer entsprechenden Darstellung abhängige, zum Beispiel elliptische, Verbreiterung einer Darstellung der Nadel in dem Überlagerungsbild kann beispielsweise intraoperativ bei der Nadelnavigation live angezeigt werden. Ein Beispiel ist eine Nadelführung, welche als augmentierte Realität einem mittels des Laparoskops 13 aufgenommenen Endoskopbild oder Laparoskopvideo überlagert wird. Hier wird beispielsweise ein entsprechender Unsicherheitsbereich im Bereich der Nadel überblendet und die Läsion virtuell angezeigt.

In einem weiteren Beispiel soll bei einer Nadelablation ein Tumor komplett innerhalb einer Ablationszone liegen. Mittels des Planungstools oder Pfad-Planungsmoduls, beispielsweise des Programmoduls 24, wird in dem Überlagerungsbild um den Tumor herum eine von der jeweiligen Ausrichtung des Nadelpfades beziehungsweise der Nadel abhängige, typischerweise anisotrope, Unsicherheitszone dargestellt, welche die Unsicherheit der Registrierung angibt oder anzeigt. Optional kann auch hier ebenso wie in den übrigen Beispielen eine räumliche Unsicherheit der Instrumentenführung und/oder der Nachverfolgungseinrichtung 12 statistisch korrekt berücksichtigt und aufaddiert werden.

Es wird in diesem Beispiel mittels des Planungsmoduls um die Nadel herum die Ablationszone visualisiert, vorzugsweise in Abhängigkeit von Parametern der jeweiligen Ablation, wie beispielsweise einer Leistung und/oder einer Dauer, und gegebenenfalls in Abhängigkeit von oder unter Berücksichtigung einer Modellierung oder Simulation eines entsprechenden Gewebes im Bereich der Ablationszone. Diese Parameter oder Parameterwerte und eine Pose, also Positionierung oder Platzierung, der Ablationsnadel werden nun so gewählt, dass möglichst der Tumor und der gesamte räumlich anisotrope Unsicherheitsbereich um den Tumor herum innerhalb der dargestellten Ablationszone liegen. Optional können hier mehrere angrenzende Ablationsvolumina geplant werden, bis die gesamte und Sicherheitszone in Summe überdeckt ist. Dies kann analog dazu gesehen werden, mehrere Nadelpfade zu planen oder vorzusehen, welche dabei nicht notwendigerweise parallel zueinander angeordnet sein oder verlaufen müssen.

In der beschriebenen Weise kann vorteilhaft eine verbesserte Unterstützung von Nadelbiopsien oder anderen instrumentellen Eingriffen oder Anwendungen, erreicht werden, wodurch gegebenenfalls weniger falsche negative Ergebnisse, insbesondere von Nadelbiopsien, entstehen können. Es werden vorteilhaft gezieltere Nadelbiopsien und andere instrumentelle Anwendungen ermöglicht. Bei gleichbleibenden oder verbesserten diagnostischen Möglichkeiten kann so vorteilhaft gegebenenfalls eine Anzahl von notwendigen Nadelpunktionen reduziert werden. Zudem wird durch das vorliegend beschriebene Verfahren eine bessere Verwendbarkeit von 2D/3D-oder 2D/2D/3D-Registrierungen zur Nadelführung ermöglicht, wodurch oftmals vorteilhaft eine Dosis für einen zusätzlichen intraoperativen 3D-Scan eingespart werden kann. Zudem wird eine gezielte Unterstützung von Ablationsprozeduren ermöglicht, wobei beispielsweise eine tatsächliche oder effektive Ablationszone mittels des Planungstools mit gegebenenfalls verbesserter Präzision vorhergesagt werden kann.

Insgesamt wird vorliegend eine Erkenntnis darüber, dass es eine Raumrichtung mit gegenüber den anderen Raumrichtungen deutlich erhöhter Registrierungsunsicherheit gibt, beispielsweise zur Endoskopausrichtung und/oder zur Planung von Instrumentenfaden ausgenutzt, um eine verbesserte Visualisierung und gegebenenfalls einen Patientennutzen durch verbesserte, insbesondere präzisere, Instrumentenführung zu erzielen. Ein jeweiliges Endoskop und/oder beispielsweise ein jeweiliges Röntgengerät können dabei so ausgerichtet werden, dass möglichst wenige Registrierungsunsicherheiten oder daraus resultierende Visualisierungsfehler für einen jeweiligen Betrachter wahrnehmbar werden.

## Patentansprüche

1. Verfahren (11) zum Unterstützen eines Anwenders, mit den Verfahrensschritten
- Bereitstellen eines ein Zielobjekt (6) abbildenden 3D-Datensatzes,
- Erfassen wenigstens eines 2D-Bildes des Zielobjekts (6),
- automatische 2D/3D-Registrierung des wenigstens einen 2D-Bildes mit dem bereitgestellten 3D-Datensatz,
- automatisches Bestimmen einer Raumrichtung (4), in welcher die 2D/3D-Registrierung eine größte Unsicherheit aufweist, und
- automatisches Erzeugen und Ausgeben eines Signals zum Unterstützen eines Ausrichtens eines zum Untersuchen des Zielobjekts (6) vorgesehenen Instruments (13) in Abhängigkeit von der bestimmten Raumrichtung (4) zum Unterstützen des Anwenders.

2. Verfahren (11) nach Anspruch 1, **dadurch gekennzeichnet, dass**
- als das Instrument (13) ein Endoskop (13) verwendet wird,
- eine Pose des Endoskops (13) in einem Koordinatensystem des 2D-Bildes automatisch nachverfolgt wird,
- aus wenigstens einem mittels des Endoskops (13) aufgenommenen Endoskopbild des Zielobjekts (6) und dem 3D-Datensatz wenigstens ein Überlagerungsbild erzeugt wird,
- als das Signal ein Hinweis an den Anwender ausgegeben wird, dass und wie der Anwender die Ausrichtung des Endoskops (13) verändern kann, sodass sich eine Erfassungsrichtung (15) des Endoskops (13) entlang der bestimmten Raumrichtung (4) erstreckt, um einen durch die Unsicherheit der 2D/3D-Registrierung verursachten Visualisierungsfehler in dem Überlagerungsbild zu verringern.

3. Verfahren (11) nach einem der vorhergehenden Ansprüche, **dadurch gekennzeichnet, dass**
- als das Instrument (13) ein mittels eines Roboters geführtes Endoskop (13) verwendet wird,
- eine Pose des Endoskops (13) in einem Koordinatensystem des 2D-Bildes automatisch nachverfolgt wird,
- aus einem mittels des Endoskops (13) aufgenommenen Endoskopbild des Zielobjekts (6) und dem 3D-Datensatz ein Überlagerungsbild erzeugt wird,
- als das Signal ein Steuersignal für den Roboter erzeugt wird, durch welches der Roboter das Endoskop (13) automatisch ausrichtet, sodass dessen Erfassungsrichtung (15) sich entlang der bestimmten Raumrichtung (4) erstreckt, um einen durch die Unsicherheit der 2D/3D-Registrierung verursachten Visualisierungsfehler in dem Überlagerungsbild zu verringern.

4. Verfahren (11) nach einem der vorhergehenden Ansprüche, **dadurch gekennzeichnet, dass**
- als das Instrument (13) ein Endoskop (13) verwendet wird,
- eine Pose des Endoskops (13) in einem Koordinatensystem des 2D-Bildes automatisch nachverfolgt wird,
- aus einem mittels des Endoskops (13) aufgenommenen Endoskopbild des Zielobjekts (6) und dem 3D-Datensatz ein Überlagerungsbild erzeugt wird,
- als das Signal ein Steuersignal für eine zum Aufnehmen des 2D-Bildes verwendete Bildgebungsmodalität (1) erzeugt wird, deren Aufnahmerichtung (4) dadurch automatisch entlang einer Erfassungsrichtung (15) des Endoskops (13) ausgerichtet wird, um einen durch die Unsicherheit der 2D/3D-Registrierung verursachten Visualisierungsfehler in dem Überlagerungsbild zu verringern.

5. Verfahren (11) nach einem der vorhergehenden Ansprüche, **dadurch gekennzeichnet, dass**
- als das Instrument (13) ein Endoskop (13) verwendet wird,
- eine Pose des Endoskops (13) in einem Koordinatensystem des 2D-Bildes automatisch nachverfolgt wird,
- aus einem mittels des Endoskops (13) aufgenommenen Endoskopbild des Zielobjekts (6) und dem 3D-Datensatz ein Überlagerungsbild erzeugt wird,
- als das Signal eine Visualisierung des Überlagerungsbildes ortsabhängig gemäß einem, insbesondere räumlich anisotropen, Grad der Unsicherheit der 2D/3D-Registrierung angepasst wird, insbesondere durch Unscharfzeichnen, Ausgrauen, Strecken und/oder Ausblenden eines Teils des Überlagerungsbildes.

6. Verfahren (11) nach Anspruch 5, **dadurch gekennzeichnet, dass** die Visualisierung des Überlagerungsbildes nur in der bestimmten Raumrichtung (4) der größten Unsicherheit der 2D/3D-Registrierung angepasst wird.

7. Verfahren (11) nach einem der vorhergehenden Ansprüche, **dadurch gekennzeichnet, dass**
- als das Instrument (13) ein Stereo-Endoskop (13) verwendet wird,
- eine Pose des Stereo-Endoskops (13) in einem Koordinatensystem des 2D-Bildes automatisch nachverfolgt wird,
- aus einem mittels des Stereo-Endoskops aufgenommenen stereoskopischen Endoskopbild des Zielobjekts (6) und dem 3D-Datensatz ein Überlagerungsbild erzeugt wird,
- mittels einer Vergenz oder Parallaxe zwischen einem linken und einem rechten Teilbild des stereoskopischen Endoskopbilds in dem Überlagerungsbild eine Raumwirkung in der bestimmten Raumrichtung (4) erzeugt wird.

8. Verfahren (11) nach einem der vorhergehenden Ansprüche, **dadurch gekennzeichnet, dass** automatisch ein Pfad bestimmt und als das Signal ausgegeben wird, entlang welchem das Instrument (13) zu führen ist, um unter Berücksichtigung der Unsicherheit der 2D/3D-Registrierung ein vorgegebenes Ziel zu erreichen.

9. Verfahren (11) nach Anspruch 8, **dadurch gekennzeichnet, dass** zum Bestimmen des Pfades eine Vorzugsrichtung einer Wirkung des Instruments (13) berücksichtigt wird.

10. Verfahren (11) nach einem der vorhergehenden Ansprüche, **dadurch gekennzeichnet, dass** das Instrument (13) in dem Überlagerungsbild dargestellt wird, wobei als oder durch das Signal die Darstellung des Instruments (13) und/oder eines Wirkungsbereiches des Instruments (13)in dem Überlagerungsbild in Abhängigkeit von der jeweiligen lokalen Unsicherheit der 2D/3D-Registrierung räumlich anisotrop verbreitert wird.

11. Verfahren (11) nach einem der vorhergehenden Ansprüche, **dadurch gekennzeichnet, dass** in Abhängigkeit von der richtungsabhängigen Unsicherheit der 2D/3D-Registrierung, insbesondere unter Berücksichtigung einer Vorzugsrichtung einer Wirkung des Instruments (13), automatisch wenigstens eine Einsatzstelle des Instruments (13) bestimmt und als das Signal ein entsprechender Hinweis ausgegeben wird.

12. Verfahren (11) nach einem der vorhergehenden Ansprüche, **dadurch gekennzeichnet, dass**
- automatisch eine jeweils aktuelle Ausrichtung des Instruments erfasst wird,
- in Abhängigkeit von der erfassten Ausrichtung des Instruments und der Unsicherheit der 2D/3D-Registrierung automatisch eine Wahrscheinlichkeit dafür berechnet wird, dass mittels des Instruments (13) in der erfassten Ausrichtung ein vorgegebenes Ziel erreicht wird, und
- die berechnete Wahrscheinlichkeit als das Signal ausgegeben wird.

13. Computerprogrammprodukt umfassend ein Computerprogramm (11), welches ein Verfahren (11) nach einem der vorhergehenden Ansprüche kodiert und welches ausgebildet und eingerichtet ist
- zum Laden in eine Speichereinrichtung (9) einer Datenverarbeitungseinrichtung (7), insbesondere eines bildgebenden Systems (1), und
- zum Ausführen des Verfahrens (11), wenn das Computerprogramm (11) mittels der Datenverarbeitungseinrichtung (7) ausgeführt wird.

14. Datenträger (9), insbesondere für ein bildgebendes System (1), auf welchem ein mittels einer Datenverarbeitungseinrichtung (7) ausführbarer Programmcode gespeichert ist, welcher ein Computerprogramm (11) umfasst, welches ein Verfahren (11) nach einem der Ansprüche 1 bis 12 kodiert, um eine bei einer Ausführung des Programmcodes durch die Datenverarbeitungseinrichtung (7) eine Ausführung des Verfahrens (11) zu bewirken.

15. Bildgebendes System mit
- einer Erfassungseinrichtung (1) zum Erfassen eines ein Zielobjekt (6) abbildenden 3D-Datensatzes und wenigstens eines 2D-Bildes des Zielobjekts (6),
- einer Datenverarbeitungseinrichtung (7) mit einer Prozessoreinrichtung (10) und einem mit dieser verbundenen Datenträger (9) nach Anspruch 14, und
- einer Ausgabeeinrichtung (17) zum Ausgeben eines automatisch erzeugten Signals.

## Claims

1. Method (11) for supporting a user, comprising the method steps:
- providing a 3D data set which depicts a target object (6),
- acquiring at least one 2D image of the target object (6),
- automatically 2D/3D registering the at least one 2D image with the 3D data set provided,
- automatically specifying a spatial direction (4) in which the 2D/3D registration exhibits greatest uncertainty, and
- as a function of the specified spatial direction (4), automatically generating and outputting a signal for the purpose of supporting an alignment of an instrument (13) that is provided for the purpose of examining the target object (6), in order to support the user.

2. Method (11) according to claim 1, **characterised in that**
- an endoscope (13) is used as the instrument (13),
- a pose of the endoscope (13) in a coordinate system of the 2D image is automatically tracked,
- at least one overlay image is generated from at least one endoscope image of the target object (6), as recorded by the endoscope (13), and the 3D data set,
- an indication to the user is output as the signal, indicating that and how the user can change the alignment of the endoscope (13) such that an acquisition direction (15) of the endoscope (13) extends along the specified spatial direction (4) in order to reduce a visualisation error, caused by the uncertainty of the 2D/3D registration, in the overlay image.

3. Method (11) according to one of the preceding claims, **characterised in that**
- an endoscope (13) guided by a robot is used as the instrument (13),
- a pose of the endoscope (13) in a coordinate system of the 2D image is automatically tracked,
- an overlay image is generated from an endoscope image of the target object (6), as recorded by the endoscope (13), and the 3D data set,
- a control signal for the robot is generated as the signal, and is used by the robot to automatically align the endoscope (13) such that the acquisition direction (15) thereof extends along the specified spatial direction (4) in order to reduce a visualisation error, caused by the uncertainty of the 2D/3D registration, in the overlay image.

4. Method (11) according to one of the preceding claims, **characterised in that**
- an endoscope (13) is used as the instrument (13),
- a pose of the endoscope (13) in a coordinate system of the 2D image is automatically tracked,
- an overlay image is generated from an endoscope image of the target object (6), as recorded by the endoscope (13), and the 3D data set,
- a control signal for an imaging modality (1) that is used to record the 2D image is generated as the signal, whereby the recording direction (4) of said imaging modality (1) is automatically aligned along an acquisition direction (15) of the endoscope (13) in order to reduce a visualisation error, caused by the uncertainty of the 2D/3D registration, in the overlay image.

5. Method (11) according to one of the preceding claims, **characterised in that**
- an endoscope (13) is used as the instrument (13),
- a pose of the endoscope (13) in a coordinate system of the 2D image is automatically tracked,
- an overlay image is generated from an endoscope image of the target object (6), as recorded by the endoscope (13), and the 3D data set,
- as the signal, a visualisation of the overlay image is adapted in a positionally dependent manner according to a degree, in particular spatially anisotropic, of the uncertainty of the 2D/3D registration, in particular by means of blurring, dimming, stretching and/or fading out a part of the overlay image.

6. Method (11) according to claim 5, **characterised in that** the visualisation of the overlay image is only adapted in the specified spatial direction (4) of greatest uncertainty of the 2D/3D registration.

7. Method (11) according to one of the preceding claims, **characterised in that**
- a stereo endoscope (13) is used as the instrument (13),
- a pose of the stereo endoscope (13) in a coordinate system of the 2D image is automatically tracked,
- an overlay image is generated from a stereoscopic endoscope image of the target object (6), as recorded by the stereo endoscope, and the 3D data set,
- a spatial effect in the specified spatial direction (4) is generated in the overlay image by means of a vergence or parallax between a left-hand part-image and a right-hand part-image of the stereoscopic endoscope image.

8. Method (11) according to one of the preceding claims, **characterised in that** a path along which the instrument (13) must be guided in order to reach a predetermined target is automatically specified and output as the signal, taking into consideration the uncertainty of the 2D/3D registration.

9. Method (11) according to claim 8, **characterised in that** a preferred direction of an effect of the instrument (13) is taken into consideration for the purpose of specifying the path.

10. Method (11) according to one of the preceding claims, **characterised in that** the instrument (13) is represented in the overlay image, wherein as or by means of the signal the representation of the instrument (13) and/or a range of effect of the instrument (13) in the overlay image is broadened in a spatially anisotropic manner as a function of the respective local uncertainty of the 2D/3D registration.

11. Method (11) according to one of the preceding claims, **characterised in that** as a function of the direction-dependent uncertainty of the 2D/3D registration, in particular taking into consideration a preferred direction of an effect of the instrument (13), at least one deployment point of the instrument (13) is specified automatically and a corresponding indication is output as the signal.

12. Method (11) according to one of the preceding claims, **characterised in that**
- a respectively current alignment of the instrument is automatically acquired,
- as a function of the acquired alignment of the instrument and the uncertainty of the 2D/3D registration, a probability that a predetermined target is reached by the instrument (13) in the acquired alignment is automatically calculated, and
- the calculated probability is output as the signal.

13. Computer program product, comprising a computer program (11) which encodes a method (11) according to one of the preceding claims and which is designed and configured
- to be loaded into a storage device (9) of a data processing device (7), in particular an imaging system (1), and
- to execute the method (11) when the computer program (11) is executed by the data processing device (7).

14. Data medium (9), in particular for an imaging system (1), on which is stored a program code that can be executed by a data processing device (7), said program code comprising a computer program (11) which encodes a method (11) according to one of claims 1 to 12, in order to effect an execution of the method (11) when the program code is executed by the data processing device (7).

15. Imaging system, comprising
- an acquisition device (1) for acquiring a 3D data set, which depicts a target object (6), and at least one 2D image of the target object (6),
- a data processing device (7) comprising a processor device (10) and a data medium (9), according to claim 14, which is connected thereto, and
- an output device (17) for outputting an automatically generated signal.

## Revendications

1. Procédé (11) pour prêter assistance à un utilisateur, comprenant les stades de procédé
- on se procure un ensemble de données en 3D reproduisant un objet (6) cible,
- on prend au moins une image en 2D de l'objet (6) cible,
- on enregistre automatiquement en 2D/3D la au moins une image en 2D avec l'ensemble de données en 3D que l'on s'est procuré,
- on détermine automatiquement une direction (4) de l'espace, dans laquelle l'enregistrement en 2D/3D a l'incertitude la plus grande, et
- on produit et on émet automatiquement un signal, pour venir à l'appui d'une orientation d'un instrument (13), prévu pour l'examen de l'objet cible, en fonction de la direction (4) de l'espace déterminé, afin de prêter assistance à l'utilisateur.

2. Procédé (11) suivant la revendication 1, **caractérisé en ce que**
- on utilise un endoscope (13) comme instrument (13),
- on suit automatiquement une pose de l'endoscope (13) dans un système de coordonnées de l'image en 2D,
- à partir d'au moins une image d'endoscope prise au moyen de l'endoscope (13), de l'objet (6) cible et de l'ensemble de données en 3D, on produit au moins une image de superposition,
- on émet comme signal une indication à l'utilisateur que et comment l'utilisateur peut modifier l'orientation de l'endoscope (13), de manière à ce qu'une direction (15) de détection de l'endoscope (13) s'étende suivant la direction (4) de l'espace déterminée, afin de diminuer, dans l'image de superposition, une erreur de visualisation provoquée par l'incertitude de l'enregistrement en 2D/3D.

3. Procédé (11) suivant l'une des revendications précédentes, **caractérisé en ce que**
- on utilise comme instrument (13) un endoscope (13) guidé au moyen d'un robot,
- on suit automatiquement une pose de l'endoscope (13) dans un système de coordonnées de l'image en 2D,
- à partir d'une image d'endoscope, prise au moyen de l'endoscope (13), de l'objet (6) cible et de l'ensemble de données en 3D, on produit une image de superposition,
- on produit comme signal un signal de commande du robot, par lequel le robot oriente automatiquement l'endoscope (13), de manière à ce que sa direction (15) de détection s'étende suivant la direction (4) de l'espace déterminée, afin de diminuer, dans l'image de superposition, des erreurs de visualisation provoquées par l'incertitude de l'enregistrement en 2D/3D.

4. Procédé (11) suivant l'une des revendications précédentes, **caractérisé en ce que**
- on utilise un endoscope (13) comme instrument (13),
- on suit automatiquement une pose de l'endoscope (13) dans un système de coordonnées de l'image en 2D,
- à partir d'une image d'endoscope, prise au moyen de l'endoscope (13), de l'objet (6) cible et de l'ensemble de données en 3D, on produit une image de superposition,
- on produit comme signal, un signal de commande d'une modalité (1) d'imagerie utilisée pour la prise de l'image en 2D, dont la direction (4) de prise est ainsi orientée automatiquement suivant une direction (15) de détection de l'endoscope (13), afin de diminuer, dans l'image de superposition, des erreurs de visualisation provoquées par l'incertitude de l'enregistrement en 2D/3D.

5. Procédé (11) suivant l'une des revendications précédentes, **caractérisé en ce que**
- on utilise un endoscope (13) comme instrument (13),
- on suit automatiquement une pose de l'endoscope (13) dans un système de coordonnées de l'image en 2D,
- à partir d'une image d'endoscope, prise au moyen de l'endoscope (13), de l'objet (6) cible et de l'ensemble de données en 3D, on produit une image de superposition,
- on adapte comme signal une visualisation de l'image de superposition en fonction de l'emplacement, suivant un degré, notamment anisotrope dans l'espace, de l'incertitude de l'enregistrement en 2D/3D, notamment par marquage de manque de netteté, grisé, étirage et/ou effacement d'une partie de l'image de superposition.

6. Procédé (11) suivant la revendication 5, **caractérisé en ce que** l'on adapte la visualisation de l'image de superposition seulement dans la direction (4) de l'espace déterminée de l'incertitude la plus grande de l'enregistrement en 2D/3D.

7. Procédé (11) suivant l'une des revendications précédentes, **caractérisé en ce que**
- on utilise un stéréo-endoscope (13) comme instrument (13),
- on suit automatiquement une pose du stéréo-endoscope (13) dans un système de coordonnées de l'image en 2D,
- à partir de l'image d'endoscope stéréoscopique, prise au moyen du stéréo-endoscope, de l'objet (6) cible et de l'ensemble de données en 3D, on produit une image de superposition,
- au moyen d'une vergence ou d'un parallaxe entre une image partielle gauche et une image partielle droite de l'image d'endoscope stéréoscopique, on produit dans l'image de superposition un effet d'espace dans la direction (4) de l'espace déterminée.

8. Procédé (11) suivant l'une des revendications précédentes, **caractérisé en ce que** l'on détermine automatiquement et on émet comme signal un trajet, le long duquel il faut guider l'instrument (13) pour, en tenant compte de l'incertitude de l'enregistrement en 2D/3D, atteindre une cible déterminée à l'avance.

9. Procédé (11) suivant la revendication 8, **caractérisé en ce que**, pour déterminer le trajet, on prend en compte une direction préférée d'un effet de l'instrument (13).

10. Procédé (11) suivant l'une des revendications précédentes, **caractérisé en ce que** l'on représente l'instrument (13) dans l'image de superposition, dans lequel, comme ou par le signal, on élargit, de manière anisotrope dans l'espace, la représentation de l'instrument (13) et/ou une plage d'effet de l'instrument (13) dans l'image de superposition en fonction de l'incertitude locale respective de l'enregistrement en 2D/3D.

11. Procédé (11) suivant l'une des revendications précédentes, **caractérisé en ce qu'**en fonction de l'incertitude, qui dépend de la direction de l'enregistrement en 2D/3D, on détermine, notamment en tenant compte d'une direction préférée d'un effet de l'instrument (13), automatiquement au moins un point d'application de l'instrument (13) et on émet comme signal une indication correspondante.

12. Procédé (11) suivant l'une des revendications précédentes, **caractérisé en ce que**
- on détecte automatiquement une orientation en cours de l'instrument,
- en fonction de l'orientation détectée de l'instrument et de l'incertitude de l'enregistrement en 2D/3D, on calcule automatiquement une probabilité d'atteindre, au moyen de l'instrument (13), dans l'orientation détectée, une cible donnée à l'avance, et
- on émet comme signal la probabilité calculée.

13. Produit de programme d'ordinateur comprenant un programme (11) d'ordinateur, qui code un procédé (11) suivant l'une des revendications précédentes et qui est constitué et conçu
- pour le chargement, dans un dispositif (9) de mémoire, d'un dispositif (7) de traitement de données, notamment d'un système (1) d'imagerie, et
- pour la réalisation du procédé (11), lorsque le programme (11) d'ordinateur est exécuté au moyen du dispositif (7) de traitement de données.

14. Support (9) de données, notamment pour un système (1) d'imagerie, sur lequel est mis en mémoire un code de programme, qui peut être réalisé au moyen d'un dispositif (7) de traitement de données et qui comprend un programme (11) d'ordinateur, qui code un procédé (11) suivant l'une des revendications 1 à 12 pour provoquer, lors d'une réalisation du code de programme par le dispositif (7) de traitement de données, une exécution du procédé (11).

15. Système d'imagerie, comprenant
- un dispositif (1) de détection pour la détection d'un ensemble de données en 3D reproduisant un objet (6) cible et d'au moins une image en 2D de l'objet (6) cible,
- un dispositif (7) de traitement de données ayant un dispositif (10) de processeur et un support (9) de données suivant la revendication 14 relié à celui-ci, et
- un dispositif (17) d'émission pour l'émission d'un signal produit automatiquement.
